# FASCICULE DE BREVET EUROPEEN

(11) **EP 4 161 434 B1**
(45) Date de publication et mention de la délivrance du brevet: **10.09.2025**
(21) Numéro de dépôt: 21730618.2
(22) Date de dépôt: 09.06.2021
(51) Int. Cl.: A61C 7/00, A61C 19/04, A61B 5/103, G06T 7/11

(54) **PROCEDE DE SUIVI D'UN MOUVEMENT DENTAIRE**
VERFAHREN ZUR VERFOLGUNG EINER ZAHNBEWEGUNG
METHOD FOR TRACKING A DENTAL MOVEMENT

(30) Priorité: 09.06.2020 FR 2006015
(43) Date de publication de la demande: 12.04.2023
(73) Titulaire: Dental Monitoring, 75017 Paris (FR)
(72) Inventeur: PELLISSARD, Thomas, 75004 PARIS (FR); GHYSELINCK, Guillaume, 59169 CANTIN (FR)
(74) Mandataire: Novagraaf Group
(86) Numéro de dépôt international: PCT/EP2021/065401
(87) Numéro de publication internationale: WO 2021/250065

(56) Documents cités:
- EP-A1- 3 432 217
- YOUNG-JUN YU: "Machine Learning for Dental Image Analysis", 29 November 2016 (2016-11-29), XP055430578, Retrieved from the Internet <URL:arXiv.org>stat>arXiv:1611.09958>

## Description

### Domaine technique

La présente invention concerne un procédé d'entrainement d'un réseau de neurones destiné à l'analyse d'une situation dentaire d'un patient, un procédé d'analyse d'une situation dentaire d'un patient mettant en œuvre le réseau de neurones ainsi entraîné, et un procédé de détermination d'une quantité de mouvement d'un organe dentaire, en particulier pour suivre l'activité d'un appareil orthodontique actif ou une perte d'efficacité d'un appareil orthodontique passif.

### Etat de la technique

Le demandeur a développé des procédés permettant le suivi à distance de la situation dentaire d'un patient, avant, pendant ou après un traitement orthodontique. Ces procédés reposent sur la comparaison de photos prises par le patient, à un instant actualisé, au moyen de son téléphone mobile, avec des vues d'un modèle numérique tridimensionnel d'au moins une de ses arcades dentaires.

Plus précisément, un modèle initial de l'arcade dentaire est réalisé à un instant initial, classiquement avec un scanner 3D, puis découpé en modèles de dent. Après que le patient a acquis les photos, le modèle initial est déformé, par déplacement des modèles de dent, pour correspondre au mieux aux photos. La comparaison des modèles initial et du modèle déformé ainsi obtenu fournit alors une information sur le déplacement des dents depuis l'instant initial. Le modèle initial étant très précis, il en est avantageusement de même pour le modèle déformé, et donc pour l'information résultant de ladite comparaison.

Ces procédés sont notamment décrits dans PCT/EP2015/074868 ou PCT/EP2015/074859.

EP 3 432 217 A1 et Young-Jun Yu: "Machine Learning for Dental Image Analysis", 29 novembre 2016, divulguent également des procédés d'entraînement d'un réseau de neurones destiné à l'analyse d'une situation dentaire d'un patient.

Ils nécessitent des ressources informatiques élevées, notamment pour déplacer les modèles de dent. Typiquement, plusieurs heures de traitement informatique sont nécessaires pour évaluer une situation dentaire.

Par ailleurs, ces procédés nécessitent la réalisation du modèle initial, et donc un déplacement du patient chez un orthodontiste, puis la mise en œuvre d'un scanner 3D. Cette procédure est coûteuse et peut être désagréable pour le patient.

Il existe donc un besoin pour un procédé permettant d'assurer un suivi à distance d'un patient et qui ne présente pas les inconvénients mentionnés ci-dessus.

Un objectif de la présente invention est de répondre, au moins partiellement, à ces besoins.

### Résumé de l'invention

La présente invention a trait à un procédé d'entraînement d'un réseau de neurones destiné à l'analyse d'une situation dentaire d'un patient actualisé selon la revendication 1, à un procédé d'analyse d'une situation dentaire d'un patient dit « patient actualisé », à un instant actualisé, selon la revendication 6, à un programme d'ordinateur comprenant des instructions de codes pour mettre en œuvre les étapes d'un procédé de détermination d'une quantité de mouvement entre un instant actualisé « antérieur » et un instant actualisé « postérieur » postérieur à l'instant actualisé antérieur, selon la revendication 12, et à l'utilisation d'un procédé d'analyse selon l'une quelconque des revendications 6 à 11, telle que définie par la revendication 16. Des manières préférentielles de réaliser cette invention sont définies par les revendications dépendantes.

L'invention propose un procédé d'entrainement d'un réseau de neurones destiné à l'analyse d'une situation dentaire d'un patient actualisé, ledit procédé comportant les étapes suivantes :
A) création d'une base d'apprentissage historique relative à un organe dentaire, par exemple la dent n°13, et à un attribut spatial associé à l'organe dentaire, par exemple la position du barycentre de la dent n°13,
   la base d'apprentissage historique comportant plus de 1 000 enregistrements historiques, chaque enregistrement historique, relatif à un patient historique respectif, comportant :
   - un ensemble d'images historiques représentant toutes ledit organe dentaire chez ledit patient historique, dit « organe dentaire historique » ; et
   - une information spatiale comportant, pour le patient historique, un ensemble de valeurs pour ledit attribut spatial, dite « information spatiale historique », à savoir, dans l'exemple considéré, des valeurs déterminant la position du barycentre de la dent n°13 pour le patient historique ;
B) entraînement du réseau de neurones, en lui fournissant en entrée lesdits ensembles d'images historiques et en sortie lesdites informations spatiales historiques.

Après l'entrainement, le réseau de neurones est ainsi capable de déterminer une information spatiale actualisée pour un ensemble d'images actualisées, compatible avec les ensembles d'images historiques de la base d'apprentissage historique, et qu'on lui présente en entrée. De préférence, la base d'apprentissage historique est spécialisée pour un organe dentaire précis et pour un attribut spatial présentant peu de variables, ce qui améliore ses performances. De préférence, le nombre d'images historiques d'un enregistrement historique est cependant limité, ce qui permet d'exploiter ensuite le réseau de neurones spécialisé sans devoir lui soumettre beaucoup d'images.

Un procédé d'entrainement selon l'invention présente de préférence une ou plusieurs des caractéristiques optionnelles suivantes :
- le réseau de neurones est convolutif (CNN, en anglais « Convolutional neural network ») ;
- l'organe dentaire est une dent ou un ensemble de moins de 5 dents, de préférence de moins de 4 dents ;
- l'organe dentaire historique est une dent ayant un numéro prédéterminé ou un ensemble de dents comportant une dent ayant un numéro prédéterminé et une ou deux dents adjacentes à ladite dent.
- l'attribut spatial comporte moins de 30, de préférence moins de 20, de préférence moins de 10 variables ;
- l'ensemble d'images historiques de tout enregistrement historique comporte plus de deux, de préférence plus de trois, de préférence plus de 5, de préférence plus de 6 et/ou moins de 30, de préférence moins de 20, de préférence moins de 15, de préférence moins de 10 images historiques ;
- chaque image historique de l'ensemble d'images historiques de tout enregistrement historique est acquise suivant une angulation choisie dans un groupe d'angulations potentielles, ou « angulations type », comportant plus de 2, de préférence plus de 4, de préférence plus de 4 et/ou moins de 30, de préférence moins de 20, de préférence moins de 10 angulations potentielles
- au moins trois images historiques de tout ensemble d'images historiques, de préférence toutes les images historiques de tout ensemble d'images historiques, sont acquises suivant des angulations différentes ;
- les images historiques sont des photographies en couleurs réelles, représentant une scène dentaire telle que pourrait la percevoir un œil humain, et/ou ne représentent pas un écarteur dentaire, et/ou sont extra-orales ;
- les images historiques sont acquises à des distances variables des patients historiques, puis croppées autour de l'organe dentaire qu'elles représentent.

L'invention concerne ainsi un procédé d'analyse d'une situation dentaire d'un patient dit « patient actualisé », à un instant actualisé, ledit procédé comportant les étapes suivantes :
a) avant l'instant actualisé, entrainement d'un réseau de neurones suivant un procédé d'entrainement selon l'invention ;
b) à l'instant actualisé, acquisition, au moyen d'un appareil d'acquisition d'images, d'un ensemble d'images actualisées compatible avec ledit réseau de neurones et représentant ledit organe dentaire du patient actualisé, dit « organe dentaire actualisé » ;
c) analyse de l'ensemble d'images actualisées au moyen dudit réseau de neurones de manière à obtenir une information spatiale comportant, pour le patient actualisé, un ensemble de valeurs pour ledit attribut spatial, dite « information spatiale actualisée ».

Une image d'une scène dentaire, par exemple une photo, est le résultat d'une projection de cette scène dentaire dans un plan. L'analyse de la représentation, sur l'image, d'un organe dentaire de la scène dentaire peut fournir une information spatiale si on connaît la forme de cet organe dentaire, ou si on connaît la forme d'un autre objet de la scène dentaire représenté et lié à l'organe dentaire. Généralement, la forme des organes dentaires, et en particulier des dents, d'un patient n'est cependant pas connue. Une simple analyse d'une image d'une scène dentaire ne permet donc généralement pas de déterminer une information spatiale fiable pour des organes dentaires.

Suivant le principe du scanner 3D, l'analyse de plusieurs images d'une même scène dentaire peut fournir une information spatiale précise sur un organe dentaire de ladite scène dentaire, même si la forme de cet organe dentaire n'était pas connue. La reconnaissance de l'organe dentaire sur les images et la comparaison des images nécessite cependant des opérations longues et coûteuses.

Comme on le verra plus en détail dans la suite de la description, de manière tout à fait inattendue, les inventeurs ont découvert que l'utilisation d'un réseau de neurones permet d'obtenir une information spatiale d'une très bonne précision, à partir de simples images, et en particulier de photos. En particulier, ils ont été surpris de constater que l'erreur sur l'information spatiale pouvait être inférieure à 1 mm, 0,5 mm, et même inférieure à 0,3 mm, sans avoir recours à un modèle tridimensionnel de l'arcade dentaire du patient. Une telle précision était en effet considérée, jusqu'à l'invention, comme impossible à atteindre exclusivement à partir d'images, sauf à réaliser un traitement complexe, comme avec un scanner 3D. En particulier, elle semblait impossible à atteindre avec de simples photos, par exemple prises par le patient lui-même.

Des essais ont montré que l'information spatiale actualisée est fiable même lorsque les images sont des photos prises sans précaution particulière avec un simple téléphone mobile, même lorsque le patient actualisé ne porte pas d'écarteur dentaire et sans que le téléphone mobile ait besoin d'être fixé sur un support, par exemple un trépied.

En outre, alors que plusieurs heures de traitement informatique étaient, jusqu'à présent, nécessaires pour évaluer chaque situation dentaire, un procédé selon l'invention permet d'obtenir une information spatiale actualisée en quelques secondes.

Enfin, aucun déplacement du patient actualisé chez un professionnel de soins dentaires n'est plus nécessaire. Le procédé d'analyse peut donc être mis en œuvre par toute personne disposant d'un téléphone mobile, indépendamment de tout contact avec un professionnel de soins dentaires.

Un procédé d'analyse selon l'invention présente de préférence une ou plusieurs des caractéristiques optionnelles suivantes :
- l'appareil d'acquisition d'images est un téléphone mobile ;
- les images actualisées sont des photographies en couleurs réelles, représentant une scène dentaire telle que pourrait la percevoir un œil humain ;
- le patient actualisé ne porte pas d'écarteur dentaire à l'étape b) ;
- les images actualisées sont extra-orales ;
- les images actualisées sont acquises à des distances variables du patient actualisé, puis croppées autour de l'organe dentaire qu'elles représentent.

De préférence, à l'étape a), on entraine une pluralité de réseaux de neurones avec des bases d'apprentissage historiques respectives qui différent en ce qu'elles concernent des organes dentaires différents et/ou des attributs spatiaux différents, de manière à obtenir une pluralité de réseaux de neurones spécialisés ;
à l'étape b), on acquiert des images actualisées et on génère, à partir desdites images actualisées, un ensemble d'images actualisées pour chacun desdits réseaux de neurones spécialisés ;
à l'étape c), on analyse chaque dit ensemble d'images actualisées au moyen du réseau de neurones spécialisé correspondant, de manière à obtenir une pluralité d'informations spatiales actualisées, que l'on peut globalement qualifier d'information statique.

De préférence, chaque base d'apprentissage historique concerne une dent ayant un numéro spécifique à ladite base d'apprentissage historique, ou un groupe de dents, les numéros des dents dudit groupe étant spécifiques à ladite base d'apprentissage historique.

De préférence, l'attribut spatial est le même pour toutes les bases d'apprentissage historiques.

Le procédé d'analyse permet ainsi une analyse de plusieurs organes dentaires avec, pour chaque organe dentaire, un réseau de neurones qui lui est spécialisé. Les informations spatiales actualisées sont ainsi à la fois précises et nombreuses.

L'invention ouvre ainsi un champ d'applications très large.

De manière particulièrement avantageuse, le procédé d'analyse selon l'invention peut être est mis en œuvre plusieurs fois, à des instants actualisés différents.

L'invention concerne en particulier un programme d'ordinateur pour la détermination d'une quantité de mouvement entre un instant actualisé « antérieur » et un instant actualisé « postérieur » postérieur à l'instant actualisé antérieur, ledit programme comportant les instructions pour réaliser le procédé comportant les étapes suivantes :
1) mise en œuvre d'un procédé d'analyse selon l'invention, à l'instant actualisé antérieur, de manière à obtenir une dite information spatiale actualisée « antérieure », ou plus généralement une information statique « antérieure » ;
2) mise en œuvre d'un procédé d'analyse selon l'invention, à l'instant actualisé postérieur, de manière à obtenir une dite information spatiale actualisée « postérieure », ou plus généralement une information statique « postérieure » ;
3) comparaison des informations spatiales actualisées antérieure et postérieure, ou plus généralement des informations statiques antérieure et postérieure, de manière à obtenir une quantité de mouvement entre les instants actualisés antérieur et postérieur, la comparaison pouvant en particulier consister en une différence entre l'information spatiale actualisée antérieure et l'information spatiale actualisée postérieure (ou plus généralement entre l'information statique antérieure et l'information statique postérieure), suivie optionnellement d'une division de ladite différence par l'intervalle de temps entre les instants actualisés antérieur et postérieur ;
4) de préférence, présentation de ladite quantité de mouvement, par exemple sur un écran d'ordinateur personnel ou d'un téléphone mobile, de préférence au patient actualisé et/ou à un professionnel de soins dentaires.

Le procédé de détermination selon l'invention présente de préférence une ou plusieurs des caractéristiques optionnelles suivantes :
- la quantité de mouvement définit une amplitude et/ou une vitesse du déplacement, en translation et/ou en rotation, entre les instants actualisés antérieur et postérieur, d'un ou plusieurs points de l'organe dentaire actualisé et/ou d'un ou plusieurs vecteurs reliant des points de l'organe dentaire actualisé ou reliant un ou plusieurs points de l'organe dentaire actualisé et un ou plusieurs autres points de la cavité buccale du patient actualisé ;
- à l'étape 3), on compare ladite quantité de mouvement à une valeur de seuil et, en fonction de la différence entre la quantité de mouvement et la valeur de seuil, on détermine
   - un indice d'activité d'un appareil orthodontique porté par le patient actualisé et/ou
   - un indice de conformité de la situation dentaire du patient actualisé à une situation prédéfinie, par exemple à une situation prédéfinie par un traitement orthodontique subi par le patient actualisé, ou à une situation résultant d'un traitement orthodontique subi par le patient actualisé, ou, indépendamment d'un traitement orthodontique, à une situation définie par un professionnel de soins dentaires, par exemple définie comme normale pour le patient actualisé ;
- l'indice d'activité et/ou l'indice de conformité sont présentés sous forme graphique ;
- l'indice d'activité et/ou l'indice de conformité sont présentés sur un écran, par exemple sur un écran d'ordinateur ou de téléphone mobile.

De préférence, comme décrit ci-dessus, on spécialise une pluralité de réseaux de neurones sur des dents ou des groupes de dents respectifs, chaque réseau de neurones étant par exemple spécialisé pour une dent ayant un numéro qui lui est spécifique.

Le procédé de détermination selon l'invention permet ainsi une analyse de l'évolution dans le temps de plusieurs organes dentaires avec, pour chaque organe dentaire, un réseau de neurones qui lui est spécialisé. Les quantités de mouvement sont ainsi à la fois précises et nombreuses. L'ensemble de ces quantités de mouvement peut globalement être qualifié d'information dynamique.

Dans un mode de réalisation préféré, la spécialisation est par type de dent. Par exemple, un réseau de neurones peut être spécialisé pour les canines, un autre réseau spécialisé pour les incisives, un troisième réseau de neurones spécialisé pour les molaires, etc.

La spécialisation peut être par numéro de dent. Par exemple, un réseau de neurones peut être spécialisé pour les dents n°13, un autre réseau spécialisé pour les dents n°14, un troisième réseau de neurones spécialisé pour les dents n°15, etc.

Les procédés d'analyse et de détermination selon l'invention peuvent être en particulier utilisés pour :
- détecter ou évaluer une position ou d'une forme d'une dent et/ou une évolution d'une position ou d'une forme d'une dent et/ou une vitesse d'évolution d'une position ou d'une forme d'une dent, notamment
   - en période de pré-traitement, c'est-à-dire dans une période qui précède un traitement orthodontique,
   - en dehors de tout traitement orthodontique, en particulier pour suivre l'éruption d'une dent ou pour détecter une récidive ou une position anormale d'une dent ou pour détecter une abrasion d'une dent, par exemple du fait d'un bruxisme, ou pour suivre l'ouverture ou la fermeture d'un espace entre deux ou plus de deux dents, notamment entre deux dents adjacentes, ou pour contrôler la stabilité ou la modification de l'occlusion,
   - dans le cadre d'un traitement orthodontique, en particulier pour suivre le déplacement d'une dent vers un positionnement prédéterminé, en particulier un positionnement amélioré de la dent, ou pour suivre l'éruption d'une dent, ou pour suivre l'ouverture ou la fermeture d'un espace entre deux ou plus de deux dents, notamment entre deux dents adjacentes, par exemple pour créer un espace adapté à la pose d'un implant dentaire, et/ou
- détecter ou évaluer une position ou une forme d'un appareil orthodontique, en particulier une position ou une forme anormale d'un appareil orthodontique, par exemple un décollement d'une bague ou d'une gouttière orthodontique, et/ou une évolution d'une position ou d'une forme d'un appareil orthodontique et/ou une vitesse d'évolution d'une position ou d'une forme d'un appareil orthodontique, notamment pour
   - optimiser la date de prise de rendez-vous chez un orthodontiste ou un dentiste, et/ou
   - évaluer l'efficacité d'un traitement orthodontique actif, et/ou
   - mesurer l'activité d'un appareil orthodontique actif ; et/ou
   - mesurer une perte d'efficacité d'un appareil orthodontique passif ; et/ou
- en dentisterie, et/ou
- mesurer une évolution de la forme de la denture du patient entre deux dates, par exemple en pré-traitement, en particulier entre deux dates séparées par un évènement susceptible d'avoir modifié la position et/ou la forme d'au moins une dent, par exemple séparées par l'occurrence d'un choc sur les dents ou par la mise en œuvre d'un dispositif dentaire susceptible de produire un effet indésirable, par exemple destiné au traitement de l'apnée du sommeil, ou par l'occurrence d'une greffe dans la bouche du patient, notamment d'une greffe en parodontie, et en particulier d'une greffe de gencive.

Les étapes A) et B), et/ou a) et c), et/ou 1) à 3) (hors étape b)), sont de préférence mises en œuvre par ordinateur. L'invention concerne ainsi également :
- un programme d'ordinateur comprenant des instructions de code de programme pour mettre en œuvre des étapes A) et B), et/ou a) et c), et/ou 1) à 3) (hors étape b)) ;
- un support informatique sur lequel est enregistré un tel programme, par exemple une mémoire ou un CD-ROM, et
- un ordinateur dans lequel est chargé un tel programme.

### Définitions

Par « patient », on entend toute personne pour laquelle un procédé selon l'invention est mis en œuvre, que cette personne soit malade ou non, subisse un traitement orthodontique ou non.

Un « traitement orthodontique » est tout ou partie d'un traitement destiné à modifier la configuration d'une arcade dentaire (traitement orthodontique actif) ou à maintenir la configuration d'une arcade dentaire, en particulier après la fin d'un traitement orthodontique actif (traitement orthodontique passif).

Un « appareil orthodontique » est un appareil porté ou destiné à être porté par un patient. Un appareil orthodontique peut être destiné à un traitement thérapeutique ou prophylactique, mais également à un traitement esthétique. Un appareil orthodontique peut être en particulier un appareil à arc et attaches, ou une gouttière orthodontique, ou un appareil auxiliaire du type Carrière Motion. Une telle gouttière s'étend de manière à suivre les dents successives de l'arcade sur laquelle elle est fixée. Elle définit une goulotte de forme générale en « U ». La configuration d'un appareil orthodontique peut être en particulier déterminée pour assurer sa fixation sur les dents, mais également en fonction d'un positionnement cible souhaité pour les dents. Plus précisément, la forme est déterminée de manière que, dans la position de service, l'appareil orthodontique exerce des forces tendant à déplacer les dents traitées vers leur positionnement cible (appareil orthodontique actif), ou à maintenir les dents dans ce positionnement cible (appareil orthodontique passif, ou « de contention »).

Une « situation dentaire » définit un ensemble de caractéristiques relatives à une arcade dentaire d'un patient à un instant, par exemple la position des dents, leur forme, la position d'un appareil orthodontique, etc. à cet instant. Ces caractéristiques peuvent également être relatives à la forme générale de l'arcade et/ou à son agencement par rapport à l'autre arcade dentaire du patient, en particulier dans la position « bouche fermée ».

Par « arcade » ou « arcade dentaire », on entend tout ou partie d'une arcade dentaire. Par « image d'une arcade », on entend ainsi une représentation en 2 dimensions de tout ou partie de ladite arcade.

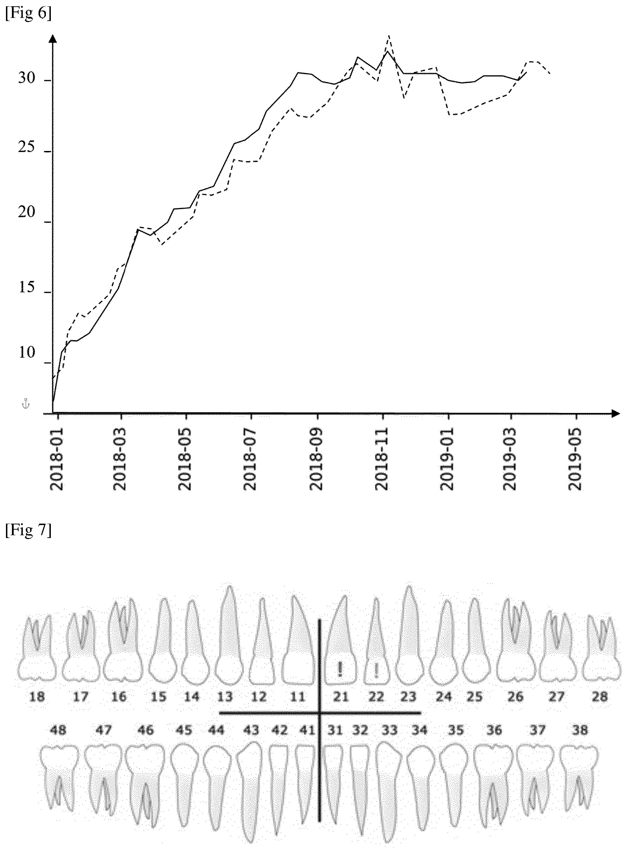

Selon la convention internationale de la Fédération Dentaire Internationale, chaque dent d'une arcade dentaire a un numéro prédéterminé. Les numéros de dent définis par cette convention sont rappelés sur la figure 7.

Une « scène » est constituée par un ensemble d'éléments qui peuvent être observés simultanément. Une « scène dentaire » est une scène comportant au moins un organe dentaire dans la cavité buccale d'un patient.

Un « point remarquable » est un point d'une scène dentaire que l'on peut identifier, par exemple le sommet de la dent ou à la pointe d'une cuspide, un point de contact interdentaire, c'est-à-dire d'une dent avec une dent adjacente, par exemple un point mésial ou distal du bord incisif d'une dent, ou un point au centre de la couronne de la dent, ou « barycentre ».

Par « organe dentaire », on entend un élément identifiable dans une cavité buccale, par exemple une dent, un ensemble de plusieurs dents, par exemple un doublet ou un triplet de dents adjacentes, une gencive ou un dispositif destiné à être porté par une arcade dentaire, et en particulier un appareil orthodontique, une couronne, un implant, un bridge, ou une facette. L'organe dentaire peut être également un sous-ensemble des éléments cités précédemment, par exemple une dent ayant un numéro déterminé, ou un ensemble de deux, trois ou plus de trois dents adjacentes, une dent dudit ensemble ayant un numéro déterminé.

Une position d'un organe dentaire est « anormale » lorsqu'elle ne respecte pas une norme thérapeutique ou esthétique.

Par "image", on entend une représentation numérique en deux dimensions, comme une photographie ou une image extraite d'un film. Une image est formée de pixels.

Une « angulation » est une orientation de l'axe optique d'un appareil d'acquisition d'images par rapport à un patient, lors de l'acquisition d'une image. Par extension, on dit qu'une image « présente » ou « a » une angulation ou est « associée à » une angulation lorsqu'elle a été acquise suivant cette angulation.

Une « zone de dent » d'une image est une partie de ladite image qui représente exclusivement une dent, c'est-à-dire qui suit le contour de cette dent sur cette image. Autrement dit, la représentation de ladite dent sur l'image représente sensiblement 100% de la zone de dent.

Par « modèle », on entend un modèle tridimensionnel numérique, dit « 3D ». Un modèle est constitué d'un ensemble de voxels. Un « modèle de dent » est un modèle numérique tridimensionnel d'une seule dent. Par « image d'une arcade » ou « modèle d'une arcade », on entend une représentation, en deux ou trois dimensions, respectivement, de tout ou partie de ladite arcade.

Un scanner 3D, ou « scanner », est un appareil bien connu permettant d'obtenir un modèle d'une dent ou d'une arcade dentaire. Il utilise classiquement une lumière structurée et, à partir de différentes images et de la mise en correspondance de points particuliers sur ces images, parvient à constituer un modèle 3D.

Les procédés selon l'invention sont mis en œuvre par ordinateur, de préférence exclusivement par ordinateur, hors l'acquisition des images. Par « ordinateur », on entend tout appareil électronique, ce qui inclut un ensemble de plusieurs machines, ayant des capacités de traitement informatique. L'ordinateur peut être un serveur à distance de l'utilisateur, par exemple être le « cloud ». De préférence, l'ordinateur est un téléphone mobile.

Classiquement, un ordinateur comporte en particulier un processeur, une mémoire, une interface homme-machine, comportant classiquement un écran, un module de communication par internet, par WIFI, par Bluetooth^{®} ou par le réseau téléphonique. Un logiciel configuré pour mettre en œuvre un procédé de l'invention est chargé dans la mémoire de l'ordinateur. L'ordinateur peut être également connecté à une imprimante.

« Premier », « deuxième », « actualisé », « historique », « », « antérieur », « postérieur », « statique », « dynamique » sont utilisés à des fins de clarté.

« Antérieur » et « postérieur » font référence des instants qui se succèdent dans le temps.

Le patient « actualisé » est le patient pour lequel on cherche à évaluer la situation dentaire. Un patient « historique » est un patient auquel correspond un enregistrement historique.

« Attribut spatial » est un terme générique qui désigne la structure d'une information spatiale. Il définit une suite ordonnée de variables dans un repère tridimensionnel, par exemple orthonormé, par exemple, pour une dent n°14 : (abscisse du barycentre ; ordonnée du barycentre ; cote du barycentre). Le repère tridimensionnel est déterminé par rapport au patient considéré, par exemple par rapport au centre de la cavité buccale du patient. Le repère tridimensionnel est de préférence fixe par rapport au patient considéré ou à une partie du patient considéré. L'origine du repère peut être en particulier au centre de la cavité buccale.

En particulier, le repère tridimensionnel est indépendant de la position et de l'orientation de l'appareil d'acquisition d'images lors de l'acquisition des images.

Une ou plusieurs valeurs d'une information spatiale peuvent être toujours nulles. Par exemple si l'attribut spatial sert à la détermination de l'abscisse d'un point remarquable de la scène dentaire, selon l'axe X du repère tridimensionnel, seule la valeur de cette abscisse n'est pas nulle. Alternativement, aucune valeur est toujours nulle.

Une information spatiale ne peut pas se déduire de la seule observation d'une image dont on ne connaît pas les conditions d'acquisition. En particulier, elle ne peut pas se déduire de la seule observation d'une unique image acquise avec un appareil d'acquisition d'images dont on ne connaît pas l'orientation et la distance par rapport à l'organe dentaire considéré, par exemple acquise avec un téléphone mobile non fixé sur un support à une distance prédéterminée du patient, par exemple en appui sur le patient, ou avec un téléphone mobile fixé sur un tel support mais dont l'orientation peut être modifiée.

En tant que telle, une image fournit de l'information « de surface », dans le plan de l'image, par exemple la position d'un point particulier d'une dent représentée sur l'image dans un référentiel bidimensionnel de l'image. Une information spatiale apporte une information de profondeur par rapport au plan de l'image. Dans l'exemple de la position d'un point particulier d'une dent représentée sur l'image, l'information spatiale fournit des coordonnées de ce point permettant de le positionner non seulement sur l'image, mais aussi dans la direction de la profondeur, perpendiculaire au plan de l'image.

L'information spatiale historique peut être relative, par exemple lorsqu'elle représente une distance entre deux points remarquables d'une dent ou entre un point remarquable de la dent et un point remarquable d'une autre dent, par exemple d'une autre dent adjacente.

Une information spatiale est une occurrence d'un attribut spatial. Par exemple (2 ; 3 ; 1,5) peut définir la position du barycentre de la dent n°14 du patient « M. Martin ». Elle est qualifiée d'« actualisée » d'ou « historique » selon qu'elle est associée à un patient actualisé ou à un patient historique.

« Antérieur » et « postérieur » font référence des instants qui se succèdent dans le temps.

« Organe dentaire » est un terme générique, qui désigne par exemple la dent n°14. « L'organe dentaire actualisé » et « l'organe dentaire historique » sont des occurrences de l'organe dentaire chez un patient actualisé et un patient historique, respectivement. Par exemple, la dent n°14 du patient « M. Martin » peut être un organe dentaire actualisé.

« Vertical », « horizontal », « droite », « gauche », « devant » ou « de face », « derrière », « au-dessus », « au-dessous » font référence à un patient qui se tient debout, verticalement.

L'information est « statique » ou « dynamique » selon qu'elle est le résultat d'une analyse à un unique instant actualisé ou qu'elle est le résultat d'analyses à plusieurs instants actualisés successifs.

Il faut interpréter "comprenant " ou "comportant " ou "présentant " de manière non restrictive, sauf indication contraire.

### Brève description des figures

D'autres caractéristiques et avantages de l'invention apparaîtront encore à la lecture de la description détaillée qui va suivre et à l'examen du dessin annexé dans lequel :
- [Fig 1] la figure 1 représente, schématiquement, un procédé dans lequel un procédé d'entrainement d'un réseau de neurones selon l'invention est mis en œuvre à plusieurs reprises pour obtenir un ensemble de réseaux de neurones chacun spécialisés sur un organe dentaire et/ou sur une information spatiale ;
- [Fig 2] la figure 2 représente, schématiquement, un procédé dans lequel un procédé d'analyse selon l'invention est mis en œuvre à plusieurs reprises avec à chaque fois un réseau de neurones spécialisé différent ;
- [Fig 3] la figure 3 représente, schématiquement, les différentes étapes d'un procédé de détermination d'une quantité de mouvement d'un organe dentaire actualisé ;
- [Fig 4] la figure 4 représente , schématiquement, un procédé dans lequel un procédé de détermination d'une quantité de mouvement selon l'invention est mis en œuvre à plusieurs reprises avec à chaque fois un réseau de neurones spécialisé différent, à deux instants différents, de manière à obtenir une information dynamique à la fois complexe et précise ;
- [Fig 5] la figure 5 est un exemple d'ensemble d'images actualisées, après un traitement pour isoler les zones de dent ;
- [Fig 6] la figure 6 est un graphique illustrant la précision d'une information spatiale actualisée obtenue selon l'invention ;
- [Fig 7] la figure 7 illustre la numérotation des dents utilisée dans le domaine dentaire ;
- [Fig 8] la figure 8 est une représentation graphique d'un indice de conformité.

Dans les différentes figures, des références identiques sont utilisées pour désigner des objets analogues ou identiques.

### Description détaillée

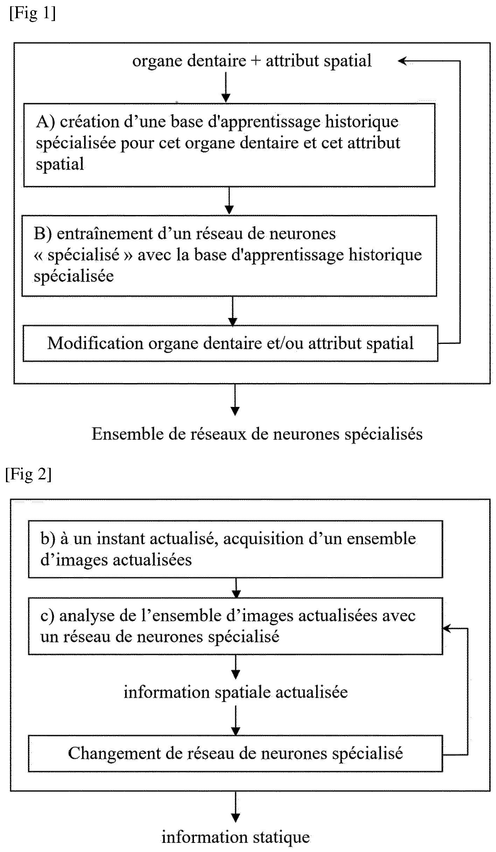

Un procédé d'entrainement selon l'invention est illustré sur la figure 1.

### Réseau de neurones

**A l'étape A),** le réseau de neurones est de préférence spécialisé dans la classification d'images.

De préférence, le réseau de neurones est un « CNN » (« Convolutional neural network »), de préférence choisi parmi les réseaux de neurones suivants :
- AlexNet (2012)
- ZF Net (2013)
- VGG Net (2014)
- GoogleNet (2015)
- Microsoft ResNet (2015)
- Caffe: BAIR Reference CaffeNet, BAIR AlexNet
- Torch
- VGG_CNN_S,VGG_CNN_M,VGG_CNN_M_2048,VGG_CNN_M_1024,V GG_CNN_M_128,VGG_CNN_F,VGG ILSVRC-2014 16-layer,VGG ILSVRC-2014 19-layer,Network-in-Network (Imagenet & CIFAR-10)
- Google : Inception (V3, V4).

De préférence, un bloc de traitement « squeeze-and-excitation (SE)», tel que décrit par Jie Hu et al, dans « Squeeze-and-Excitation Networks », arXiv:1709.01507v4 [cs.CV] 16 May 2019, est ajouté à un opérateur convolutif CNN. De préférence encore, le réseau de neurones est du type VGG avec un bloc SE.

Pour être opérationnel, le réseau de neurones d'orientation doit être classiquement entrainé par un processus d'apprentissage appelé *« deep learning »,* à partir d'une base d'apprentissage historique adaptée à la fonction souhaitée.

### Base d'apprentissage historique

L'entrainement d'un réseau de neurones est un processus bien connu de l'homme de l'art. Il consiste à le confronter à une base d'apprentissage historique contenant des enregistrements historiques contenant chacun une donnée d'entrée et une donnée de sortie.

Le réseau de neurones apprend ainsi à faire « correspondre », c'est-à-dire à connecter l'une à l'autre, les données d'entrée et de sortie.

Pour qu'il apprenne à évaluer, à partir d'un ensemble d'images actualisées, une information spatiale actualisée relative à un organe dentaire actualisé représenté sur ces images, par exemple relative à la configuration des dents représentées sur ces images, la base d'apprentissage historique est de préférence constituée d'un ensemble d'enregistrements historiques comportant chacun :
- un ensemble d'images « historiques » représentant toutes un organe dentaire « historique » d'un patient « historique », de préférence une photo représentant au moins une dent de ce patient ;
- un descriptif historique de l'ensemble d'images historiques, le descriptif comportant une information spatiale historique relative à l'organe dentaire historique représenté sur les images historiques.

De préférence, la base d'apprentissage historique comporte plus de 1000, plus de 5 000, de préférence plus de 10 000, de préférence plus de 30 000, de préférence plus de 50 000, de préférence plus de 100 000 enregistrements historiques. Plus le nombre d'enregistrements est élevé, meilleure est la capacité d'analyse du réseau de neurones. Le nombre d'enregistrements historiques est classiquement inférieur à 10 000 000 ou à 1 000 000.

Les enregistrements historiques sont chacun associés à un patient historique respectif.

### Images historiques

Les ensembles d'images historiques comportent tous le même nombre d'images historiques, quel que soit l'enregistrement historique considéré. Ce nombre est de préférence supérieur à 1, à 2, à 4, à 5 et/ou inférieur à 100, à 50, à 20, à 15 ou à 10, de préférence compris entre 5 et 15.

Dans un mode de réalisation, ce nombre est de 3, les 3 images historiques ayant des angulations différentes.

L'acquisition des images historiques est réalisée au moyen d'un appareil d'acquisition d'images, de préférence choisi parmi un téléphone mobile, un appareil photo dit « connecté », une montre dite « intelligente », ou « smartwatch », une tablette ou un ordinateur personnel, fixe ou portable, comportant un système d'acquisition d'images, comme une webcam ou un appareil photo.

Les images historiques sont de préférence des photos, de préférence prises avec un téléphone mobile.

De préférence, chaque image historique est une photographie ou est une image extraite d'un film. Elle est de préférence en couleurs, de préférence en couleurs réelles. De préférence, elle représente une scène dentaire sensiblement comme la voit l'opérateur de l'appareil d'acquisition d'images, et en particulier avec les mêmes couleurs.

Les images historiques sont de préférence extra-orales, c'est-à-dire que l'appareil d'acquisition de ces images n'est pas introduit dans la bouche du patient historique.

De préférence encore, l'appareil d'acquisition des images historiques est écarté de la bouche du patient historique de plus de 5 cm, plus de 8 cm, voire plus de 10 cm, ce qui évite la condensation de vapeur d'eau sur l'optique de l'appareil d'acquisition d'images et facilite la mise au point. En outre, de préférence, l'appareil d'acquisition d'images, en particulier le téléphone mobile, n'est pourvu d'aucune optique spécifique pour l'acquisition des images historiques, ce qui est notamment possible du fait de l'écartement entre l'appareil d'acquisition d'images et la bouche du patient historique lors de l'acquisition.

Dans un mode de réalisation, le patient historique porte un écarteur dentaire pour mieux découvrir ses dents. L'écarteur peut présenter les caractéristiques des écarteurs conventionnels. De préférence, il comporte un rebord s'étendant autour d'une ouverture d'écarteur et agencé de manière que les lèvres du patient historique puissent y reposer en laissant apparaître les dents du patient historique à travers ladite ouverture d'écarteur. De préférence, l'écarteur comporte des oreilles d'écartement des joues afin que l'appareil d'acquisition des images historiques puisse acquérir, à travers l'ouverture d'écarteur, des photos de faces vestibulaires de dents disposées au fond de la cavité buccale, comme des molaires.

Dans un mode de réalisation préféré, aucun écarteur dentaire n'est utilisé. Des essais ont en effet montré que les photos prises sans écarteur suffisent généralement à la mise en œuvre du procédé selon l'invention. Bien entendu, si nécessaire, le patient historique peut devoir écarter une joue ou une lèvre avec un doigt ou avec une cuiller, par exemple.

Toutes les images historiques d'un enregistrement historique représentent, au moins partiellement, le même organe dentaire du patient historique associé à l'enregistrement, par exemple les incisives du patient historique.

Une image historique peut en particulier représenter une ou plusieurs dents. De préférence, elle représente plusieurs dents et au moins une partie de la gencive, voire des lèvres ou du nez du patient historique

Les images historiques d'un enregistrement historique représentent toutes le même organe dentaire historique, mais de préférence suivant différentes angulations, c'est-à-dire qu'elles ont été acquises avec des orientations différentes de l'appareil d'acquisition d'images par rapport à la cavité buccale du patient historique. Par exemple, un enregistrement historique peut comporter 6 images historiques représentant une même dent « vue de face », « vue de face-droite », « vue de droite », « vue de face-gauche », « vue de gauche » et « vue de dessous », respectivement.

Les angulations des images historiques d'un enregistrement historique sont de préférence sensiblement identiques, quel que soit l'enregistrement historique considéré, pour tous les enregistrements historiques relatifs à un même organe dentaire historique, par exemple à un même type de dent, par exemple pour tous les enregistrements historiques relatifs à une incisive, ou à un même numéro de dent, par exemple pour tous les enregistrements historiques relatifs à une incisive supérieure droite.

Un enregistrement historique peut comporter une ou plusieurs images historiques selon une même angulation.

Lors de l'acquisition des images, historiques comme actualisées, d'un procédé selon l'invention, l'angulation peut être définie avec un niveau de précision qui n'est pas limitatif. Cependant, des essais ont montré que l'angulation n'a pas besoin d'être définie très précisément. Avantageusement, l'acquisition de ces images ne nécessite donc pas une formation préalable de l'opérateur de l'appareil d'acquisition d'images. Les images historiques comme les images actualisées peuvent être ainsi acquises avec peu de précaution, par exemple avec un simple téléphone mobile.

Dans un mode de réalisation préféré, les angulations sont définies de manière très générale. Par exemple, les angulations peuvent être choisies dans un groupe d' angulations potentielles constitué des angulations « vue de face », « « vue de droite », « vue de face-droite », « vue de gauche », « vue de face-gauche », « vue de dessous », « vue de face-dessous », « vue de dessus », », « vue de face-dessus ».

L'angulation peut être définie par rapport à un repère « naturel », c'est-à-dire en fonction de la façon dont le patient perçoit l'appareil d'acquisition d'images. Dans ce repère, l'angulation est par exemple choisie dans un groupe d'angulations potentielles constitué des angulations suivantes :
dans le plan occlusal
   - « vue de face » lorsque l'axe optique de l'appareil d'acquisition d'images est sensiblement confondu avec une droite à l'intersection entre le plan occlusal et le plan sagittal médian ;
   - « vue de droite » lorsque l'axe optique de l'appareil d'acquisition d'images est sensiblement dans le plan occlusal et perpendiculaire au plan sagittal médian, l'appareil d'acquisition d'images étant à droite du patient ;
   - « vue de gauche » lorsque l'axe optique de l'appareil d'acquisition d'images est sensiblement dans le plan occlusal et perpendiculaire au plan sagittal médian, l'appareil d'acquisition d'images étant à gauche du patient ;
dans le plan sagittal médian
   - « vue de dessus » lorsque l'axe optique de l'appareil d'acquisition d'images est sensiblement dans le plan sagittal médian et perpendiculaire au plan occlusal, l'appareil d'acquisition d'images étant au-dessus du patient ;
   - « vue de dessous » lorsque l'axe optique de l'appareil d'acquisition d'images est sensiblement dans le plan sagittal médian et perpendiculaire au plan occlusal, l'appareil d'acquisition d'images étant en dessous du patient.

L'angulation peut être définie plus précisément. En particulier, pour chacune des angulations ci-dessus, l'axe optique de l'appareil d'acquisition d'images est dans le plan occlusal ou le plan sagittal médian. Il est par exemple possible d'ajouter des angulations :
- dans un des deux plans « face-droite » et « face-gauche » inclinés à 45° par rapport au plan sagittal médian et contenant la droite à l'intersection entre le plan occlusal et le plan sagittal médian : vue de face-droite et de droite, vue de face-droite et de gauche, vue de face-gauche et de droite, et vue de face-gauche et de gauche, ou
- dans un des deux plans « occlusal - dessus » et « occlusal - dessous » inclinés à 45° par rapport au plan occlusal et contenant la droite à l'intersection entre le plan occlusal et le plan parallèle au plan frontal et qui passe au centre de la cavité buccale : vue occlusal - dessus et de face, ou
- dans lesquelles l'axe optique est à l'intersection entre un premier plan choisi parmi un des deux plans « face-droite » et « face-gauche » et un deuxième plan choisi parmi un des deux plans « occlusal - dessus » et « occlusal - dessous ».

De préférence, les angulations sont choisies dans un groupe d'angulations potentielles constitué des angulations listées ci-dessus.

De préférence, les angulations sont cependant déterminées en fonction de l'organe dentaire. Par exemple, si l'organe dentaire est une dent, ou un groupe de dents, les angulations sont de préférence fixées en fonction du numéro de ladite dent ou desdites dents. La configuration de la bouche ne permet pas toujours en effet d'utiliser les mêmes angulations pour toutes les dents.

Les mêmes angulations peuvent cependant parfois être utilisées pour deux organes dentaires différents, par exemple une incisive et une canine.

Un positionnement précis de l'appareil d'acquisition lors de l'acquisition des images historiques n'est pas nécessaire. Les images historiques peuvent être ainsi acquises à des distances de la bouche différentes les unes des autres. Des essais ont montré que l'organe dentaire historique, par exemple une dent, peut être représenté à une échelle différente selon l'image historique considérée et/ou selon l'enregistrement historique considéré sans que les performances du réseau de neurones entrainé en soient sensiblement affectées de manière significative.

De préférence cependant, l'appareil d'acquisition d'images est fixé sur un support qui est positionné en appui sur le corps du patient historique, de préférence introduit dans la bouche du patient historique. Lorsque le support est rigide, il impose avantageusement une distance prédéterminée entre l'appareil d'acquisition d'images et la bouche du patient historique. Les performances du réseau de neurones en sont améliorées.

De préférence, le support porte un écarteur dentaire conventionnel. Un tel écarteur dentaire comporte classiquement un rebord s'étendant autour d'une ouverture d'écarteur et agencé de manière que les lèvres du patient historique puissent y reposer en laissant apparaître les dents du patient à travers ladite ouverture d'écarteur.

De préférence, on utilise un dispositif de prise de vue tel que décrit dans la demande de brevet européen déposée le 10 octobre 2017 sous le n° 17 306361.1.

En outre, de préférence, les images historiques sont « croppées » avant d'être incorporées dans un enregistrement historique. Le « croppage », ou « redécoupage », est une opération classique qui comporte un rognage d'une image afin d'en isoler la partie pertinente, puis une normalisation des dimensions. De préférence, les images historiques sont rognées pour isoler l'organe dentaire historique, c'est-à-dire ne représenter sensiblement que l'organe dentaire historique. Le redécoupage peut être réalisé manuellement ou, comme décrit ci-après, par ordinateur, et en particulier au moyen d'un réseau de neurones entrainé à cet effet. Le redécoupage des images historiques améliore considérablement les performances du réseaux de neurones entrainé.

Des essais ont également montré que, comme indiqué précédemment, aucune angulation n'a besoin d'être fixée de manière précise.

### Organe dentaire historique et spécialisation

L'organe dentaire peut être en particulier une dent ou un ensemble de dents ou une arcade dentaire. De préférence, il est choisi de manière à limiter la variété de sa forme entre les différents enregistrements historiques. L'organe dentaire est de préférence une dent d'un type particulier, ou une dent ayant un numéro particulier.

Un organe dentaire « historique » est l'organe dentaire dans le cas particulier d'un patient historique considéré. Autrement dit, dans la base d'apprentissage historique, tous les organes dentaires historiques sont des occurrences particulières de l'organe dentaire associé à la base d'apprentissage.

De préférence, la base d'apprentissage, et donc le réseau de neurones, sont spécialisés pour les seules dents ayant un numéro particulier. Par exemple, ils sont spécialisés pour les incisives supérieures droites. Toutes les images historiques d'un enregistrement historique représentent alors toutes une même dent historique, l'information spatiale historique de cet enregistrement historique est relative à cette dent historique, et toutes les dents historiques de la base d'apprentissage portent le même numéro. Cette spécialisation du réseau de neurones pour un numéro de dent améliore considérablement son efficacité.

Plusieurs réseaux de neurones ainsi spécialisés sont de préférence entrainés, chaque réseau de neurones étant entrainé avec une base d'apprentissage historique dédiée à un type de dent ou à un numéro de dent. Avantageusement, l'analyse d'une situation dentaire peut ainsi mettre en œuvre, pour chaque dent du patient actualisé, un réseau de neurones spécialisé sur le type ou le numéro de dent correspondant.

### Descriptif historique

Un descriptif historique d'un ensemble d'images historiques comporte une information spatiale historique, c'est-à-dire un ensemble de valeurs pour les variables d'un attribut spatial, ces valeurs étant relatives à l'organe dentaire historique représenté sur les images historiques. Par définition, un attribut spatial comporte au moins trois variables correspondant aux trois dimensions de l'espace. L'information spatiale est donc un ensemble d'au moins trois valeurs pour au moins trois variables respectives de l'attribut spatial.

L'attribut spatial est le même pour toutes les images historiques de l'ensemble, mais aussi pour tous les enregistrements historiques.

Il peut en particulier définir :
- une ou plusieurs positions dans l'espace d'un ou plusieurs organes dentaires et/ou d'une ou plusieurs parties d'organe dentaire, et/ou
- une ou plusieurs directions d'orientation dans l'espace d'un ou plusieurs organes dentaires et/ou d'une ou plusieurs parties d'organe dentaire, et/ou
- un ou plusieurs sens d'orientation dans l'espace d'un ou plusieurs organes dentaires et/ou d'une ou plusieurs parties d'organe dentaire.

Ladite partie de l'organe dentaire peut être par exemple
- un ou plusieurs points, par exemple, si l'organe dentaire est une dent, un point de contact avec une dent adjacente ou le barycentre de la dent, et/ou
- une ou plusieurs lignes, par exemple, si l'organe dentaire est une dent, une ligne de séparation entre une dent adjacente ou une arête d'une cuspide de la dent, et/ou
- une ou plusieurs surfaces de cet organe dentaire.

En particulier, si l'attribut spatial (x_{A}, y_{A}, z_{A}, x_{B}, y_{B} et z_{B}) définit des positions pour deux points (x_{A}, y_{A} et z_{A}) et (x_{B}, y_{B} et z_{B}), respectivement, d'une manière ordonnée (la position du point étant A avant la position du point B), il définit indirectement une direction d'orientation, un sens d'orientation et une distance. S'il définit des positions pour trois points et que ces positions sont ordonnées, il définit indirectement trois directions d'orientation, et donc un angle entre des couples de ces directions, un sens d'orientation le long de chacune de ces directions, et trois distances.

Par exemple s'il définit la position du barycentre d'une première dent, puis la position du barycentre de la dent adjacente, à gauche de la première dent, l'attribut spatial définit, directement la position de ces barycentres, mais aussi, indirectement, la direction d'orientation de la droite qui relie ces points, un sens d'orientation, depuis le premier barycentre vers le second barycentre, et une distance entre ces barycentres.

Un attribut spatial peut définir une position absolue, dans un repère tridimensionnel immobile par rapport au patient, par exemple dont l'origine est au centre de la cavité buccale du patient considéré, l'axe des abscisses est horizontal et orienté vers l'avant, l'axe de ordonnées est horizontal et orienté vers la droite et l'axe des cotes est vertical et orienté vers le haut. Il peut aussi définir un vecteur entre deux points, c'est-à-dire une position relative d'un point par rapport à un autre un point. Par exemple, l'attribut spatial pourrait être (x_{B} - x_{A}, y_{B} - y_{A}, z_{B} - z_{A}), c'est-à-dire fournir la position du point B relativement au point A.

L'information spatiale peut ainsi définir une position, absolue ou relative, et/ou une direction d'orientation et/ou un sens d'orientation et/ou une distance dans une situation dentaire particulière.

La détermination de l'information spatiale historique d'un enregistrement historique ne pose pas de difficulté. Elle peut être déterminée par tout moyen, par exemple manuellement ou par ordinateur, notamment en effectuant des mesures sur le patient historique ou sur un moulage en plâtre de ses dents ou sur un modèle tridimensionnel numérique de l'arcade dentaire portant la dent considérée.

De préférence, l'attribut spatial définit moins de 30, de préférence moins de 20, de préférence moins de 10 variables, de préférence moins de 5 variables, de préférence moins de 4 variables. L'efficacité du réseau de neurones entrainé en est améliorée.

Dans un mode de réalisation, l'attribut spatial définit des positions dans l'espace pour 1, de préférence plus de 1, de préférence plus de 2 et/ou moins de 5, de préférence moins de 4 points remarquables de l'organe dentaire, de préférence une dent ayant un numéro particulier. Limiter l'information spatiale historique relative à une dent à un nombre réduit de points améliore considérablement l'efficacité du réseau de neurones.

**A l'étape B),** le réseau de neurones est entrainé avec la base d'apprentissage historique, en lui présentant successivement les enregistrements historiques, et plus précisément les ensembles d'images historiques en entrée et les informations spatiales historiques en sortie.

Il apprend ainsi à fournir, à sa sortie, à partir d'un ensemble d'images similaire à un ensemble d'images historiques qu'on lui présente en entrée, une information spatiale correspondante. En particulier, après avoir été ainsi entrainé, le réseau de neurones peut fournir une information spatiale « actualisée » relative à un organe dentaire « actualisé » d'un patient « actualisé », à un instant « actualisé ». L'information spatiale actualisée peut donc servir, seule ou en combinaison avec d'autres informations, à analyser d'une situation dentaire du patient actualisé. Un procédé d'analyse selon l'invention comporte les étapes a) à c), comme illustré sur la figure 2.

Le réseau de neurones ainsi entrainé est ainsi capable de déterminer une information spatiale actualisée pour un ensemble d'images actualisées, compatible avec les ensembles d'images historiques de la base d'apprentissage historique, prises sur un patient actualisé quelconque, et qu'on lui présente en entrée.

**L'étape a)** consiste à exécuter les étapes A) et B) ci-dessus.

**A l'étape b),** on acquiert, à l'instant actualisé, au moyen d'un appareil d'acquisition d'images, un ensemble d'images actualisées représentant l'organe dentaire actualisé, de préférence une dent actualisée, du patient actualisé.

L'instant actualisé peut être
- indépendant de tout traitement orthodontique, par exemple pour que chacun puisse contrôler sa situation dentaire à tout instant, avec son téléphone mobile ;
- pendant un traitement orthodontique actif ;
- après un traitement orthodontique actif, en particulier lors d'un traitement orthodontique passif.

Le procédé d'analyse peut notamment être mis en œuvre pendant un traitement orthodontique actif pour en contrôler le déroulement, l'instant actualisé étant de préférence moins de 3 mois, moins de 2 mois, et/ou plus de 1 semaine, de préférence plus de 2 semaines après la pose d'un appareil orthodontique actif, par exemple d'une gouttière orthodontique (ou « aligneur ») ou d'un arc orthodontique, destiné à corriger le positionnement des dents du patient actualisé.

Le procédé d'analyse peut être également mis en œuvre après un traitement orthodontique, pour vérifier que le positionnement des dents n'évolue pas de façon défavorable (« récidive »). L'instant actualisé est alors de préférence moins de 3 mois, moins de 2 mois, et/ou plus de 1 semaine, de préférence plus de 2 semaines après la fin du traitement orthodontique actif et la pose d'un appareil orthodontique passif destiné maintenir les dents en position, dit « attèle de contention ».

Les images actualisées sont de préférence extra-orales.

De préférence, les images actualisées sont des photographies ou des images extraites d'un film. Elles sont de préférence en couleurs, de préférence en couleurs réelles. De préférence, elles représentent l'arcade dentaire sensiblement comme la voit l'opérateur de l'appareil d'acquisition d'images.

Dans un mode de réalisation, le patient actualisé porte un écarteur dentaire pour mieux découvrir ses dents. De préférence cependant, aucun écarteur dentaire n'est utilisé. Bien entendu, si nécessaire, le patient actualisé peut devoir écarter une joue ou une lèvre avec un doigt ou avec une cuillère ou tout autre ustensile adapté à cet effet, par exemple.

Une image actualisée peut représenter une ou plusieurs dents. De préférence, elle représente au moins une partie de la gencive, voire des lèvres ou du nez du patient.

L'ensemble des images actualisées doit être adapté au réseau de neurones, c'est-à-dire compatible avec lui. Autrement dit, l'ensemble des images actualisées doit être tel qu'il aurait pu être utilisé pour un enregistrement historique.

Le nombre d'images actualisées est de préférence identique au nombre d'images historiques d'un enregistrement historique.

Les images actualisées doivent représenter le même organe dentaire que les images historiques, par exemple la dent n°14.

Les angulations des images actualisées sont de préférence similaires ou proches de celles des images historiques de tout enregistrement historique.

De manière générale, les images actualisées doivent être similaires aux images historiques ayant servi à l'entrainement du réseau de neurones. Par exemple, si ces images historiques sont des photos extraorales qui ont généralement été prises par les patients historiques eux-mêmes, à une distance variable, par exemple à une distance comprise entre 10 et 50 cm de leurs bouches, avec une angulation approximative (par exemple « vue de face » ou « vue de droite »), il est préférable que les images actualisées soient également des photos prises ces conditions d'acquisition. Si les images historiques représentent des vues prises avec un écarteur dentaire, il en est de préférence de même pour les images actualisées.

L'acquisition des images actualisées est réalisée au moyen d'un appareil d'acquisition d'images qui peut être identique ou différent, de préférence du même type que celui ayant servi à l'acquisition des images historiques.

Il est de préférence choisi parmi un téléphone mobile, un appareil photo dit « connecté », une montre dite « intelligente », ou « smartwatch », une tablette ou un ordinateur personnel, fixe ou portable, comportant un système d'acquisition d'images, comme une webcam ou un appareil photo. De préférence, l'appareil d'acquisition d'images est un téléphone mobile. De préférence, l'appareil d'acquisition d'images, en particulier le téléphone mobile, n'est pourvu d'aucune optique spécifique pour l'acquisition des images actualisées.

De préférence encore, pour acquérir une image actualisée, l'appareil d'acquisition des images actualisées est écarté de la bouche du patient actualisée de plus de 5 cm, plus de 8 cm, voire plus de 10 cm et/ou inférieure à 50 cm. Cette distance n'a avantageusement pas besoin d'être fixée précisément.

De préférence cependant, comme les images historiques, les images actualisées sont « croppées » (ou « redécoupées ») avant d'être incorporées dans l'ensemble d'images actualisées qui sera soumis au réseau de neurones entrainé avec la base d'apprentissage historique. De préférence, les images actualisées sont rognées pour isoler l'organe dentaire actualisée, c'est-à-dire ne représenter sensiblement que l'organe dentaire actualisé. Le redécoupage peut être réalisé manuellement ou, de préférence par ordinateur, et en particulier au moyen d'un réseau de neurones entrainé à cet effet.

En particulier, un réseau de neurones peut être entrainé à identifier l'organe dentaire sur des images, par exemple à identifier les zones de dent. Un tel réseau de neurones « d'identification d'organes dentaires » est décrit ci-après. Pour cropper une image actualisée, il suffit alors d'identifier l'organe dentaire actualisé avec ce réseau de neurones, de définir le plus petit rectangle pouvant contenir l'organe dentaire identifié sur cet image, de ne retenir que l'intérieur de ce rectangle pour définir une découpe, puis de normaliser les dimensions de la découpe pour définir l'image actualisée à incorporer dans l'ensemble d'images actualisées. Si le rapport longueur/largeur du rectangle est sensiblement toujours le même, quelle que soit l'image actualisée, il est probable que les conditions d'acquisition des images sont similaires, ce qui est avantageux. La normalisation des dimensions de la découpe consiste à ajuster ces dimensions pour que toutes les images actualisées aient les mêmes dimensions. De préférence, l'opération de redécoupage des images historiques est similaire à celle des images actualisées, de manière que les dimensions et le nombre de pixels de ces images soient similaires ou sensiblement identiques.

Le redécoupage des images historiques et des images actualisées améliore considérablement les performances du réseaux de neurones entrainé.

L'appareil d'acquisition d'images est utilisé par un opérateur qui est de préférence le patient actualisé ou un proche du patient actualisé, mais qui peut être toute autre personne, notamment un dentiste ou un orthodontiste ou un personnel soignant. De préférence, l'acquisition des images actualisées est effectuée par le patient actualisé.

De préférence, l'acquisition des images actualisées est effectuée sans utilisation d'un support, prenant appui sur le sol et immobilisant l'appareil d'acquisition d'images, et notamment sans trépied.

Dans un mode de réalisation cependant, l'appareil d'acquisition d'images est fixé sur un support qui est positionné en appui sur le corps du patient historique, de préférence partiellement introduit dans la bouche du patient historique. Lorsque le support est rigide, il impose avantageusement une distance prédéterminée entre l'appareil d'acquisition d'images et la bouche du patient actualisé. Les performances du réseau de neurones en sont améliorées.

De préférence, le support porte un écarteur dentaire conventionnel. Un tel écarteur dentaire comporte classiquement un rebord s'étendant autour d'une ouverture d'écarteur et agencé de manière que les lèvres du patient actualisé puissent y reposer en laissant apparaître les dents du patient actualisé à travers ladite ouverture d'écarteur.

De préférence, on utilise un dispositif de prise de vue tel que décrit dans la demande de brevet européen déposée le 10 octobre 2017 sous le n° 17 306361.1.

### Constitution de l'ensemble d'images actualisées pendant l'acquisition

De préférence encore, l'opérateur est guidé lors de l'étape b), de préférence en temps réel, pour orienter l'appareil d'acquisition d'images suivant des angulations prédéterminées, et/ou, de préférence, pour prendre un nombre d'images prédéterminé selon les différentes angulations, et/ou pour orienter l'appareil d'acquisition d'images selon les angulations requises.

A cet effet, un applicatif est de préférence chargé dans l'appareil d'acquisition d'images afin d'assurer un contrôle embarqué lors de l'étape b), c'est-à-dire de vérifier que le nombre et/ou l'angulation et/ou la qualité des images actualisées sont satisfaisants.

L'applicatif peut notamment mettre en œuvre des moyens détrompeurs facilitant le positionnement approximatif de l'appareil d'acquisition d'images par rapport au patient actualisé avant l'acquisition de l'image actualisée.

Les moyens détrompeurs peuvent en particulier comporter une référence qui apparait sur l'écran de l'appareil d'acquisition d'images et que l'opérateur doit faire correspondre, par exemple superposer, à une partie du patient actualisé affichée sur cet écran, par exemple un contour d'une dent, d'une gencive, d'une lèvre, ou du visage.

La référence peut être par exemple une forme géométrique, par exemple un point, un ou plusieurs traits, par exemple parallèles, une étoile, un cercle, un ovale, un polygone régulier, notamment un carré, un rectangle ou un losange, ou toute combinaison d'une ou plusieurs de ces formes. La ou les références sont de préférence "immobiles" sur l'écran, c'est-à-dire qu'elles ne se déplacent pas sur l'écran lorsque l'appareil d'acquisition d'images est en mouvement.

La référence peut par exemple comporter une ligne horizontale destinée à être alignée avec la direction générale de la représentation, sur l'écran, de la jointure horizontale entre les dents supérieures et les dents inférieures lorsque les dents sont serrées par le patient actualisé, et/ou une ligne verticale destinée à être alignée avec la représentation de la jointure verticale entre les deux incisives supérieures. Une référence peut être, par exemple, constituée par des deux cercles à placer au-dessus de la représentation, sur l'écran, des deux yeux du patient actualisé. Une référence peut être, par exemple, constituée par un ovale à placer autour de la représentation, sur l'écran, de la bouche ou du visage du patient actualisé.

La « partie du patient » peut être également sur un support porté par le patient actualisé, par exemple porté par un écarteur dentaire ou par une pièce mordue par le patient actualisé.

L'applicatif peut également aider l'opérateur à modifier l'angulation de l'appareil d'acquisition d'images, par exemple en annonçant ou en affichant sur l'écran des messages comme « prenez une photo de droite », « plus haut », plus bas », etc., ou en émettant une succession de bips dont la fréquence augmente à mesure que l'orientation de l'appareil d'acquisition d'images s'améliore. A cet effet, il doit analyser en temps réel l'image affichée sur l'écran de l'appareil d'acquisition d'images, en particulier pour déterminer si l'organe dentaire est représenté et, de préférence, pour vérifier si l'angulation est adaptée.

Les algorithmes de détection d'objets dans des images sont bien connus de l'homme du métier et peuvent être utilisés pour rechercher l'organe dentaire dans l'image affichée. De préférence, on utilise un réseau de neurones d'identification d'organes dentaires, de préférence choisi parmi les « Object Detection Networks », par exemple:
- R-CNN (2013)
- SSD (Single Shot MultiBox Detector : Object Detection network), Faster R-CNN (Faster Region-based Convolutional Network method : Object Detection network)
- Faster R-CNN (2015)
- SSD (2015)
- RCF (Richer Convolutional Features for Edge Detection) (2017).

L'entrainement d'un réseau de neurones pour détecter un organe dentaire, par exemple une dent ayant un numéro déterminé, dans une image ne pose aucune difficulté à l'homme de l'art. En particulier, on présente en entrée du réseau de neurones des images et en sortie une information sur la présence ou l'absence de l'organe dentaire.

Les articles suivants traitent en particulier de la détection ou de la segmentation : https://arxiv.org/pdf/1405.0312.pdf et https://arxiv.org/pdf/1703.06870.pdf.

Dans un mode de réalisation, ledit réseau de neurones est entrainé avec une base d'apprentissage constituée d'un ensemble de plus de 1000, de préférence plus de 10 000 enregistrements comportant chacun :
- une image comportant une zone représentant l'organe dentaire, par exemple comportant au moins une zone de dent relative à une dent ayant un numéro déterminé ;
- un descriptif de ladite image identifiant la zone représentant l'organe dentaire sur ladite image.

Lors de l'entrainement, chaque image est fournie à l'entrée du réseau de neurones alors que le descriptif associé est fourni à la sortie de réseau de neurones.

A l'issue dudit entrainement, le réseau de neurones est ainsi apte à de déterminer une zone représentant l'organe dentaire, par exemple une zone de dent, dans une image qu'on lui fournit en entrée.

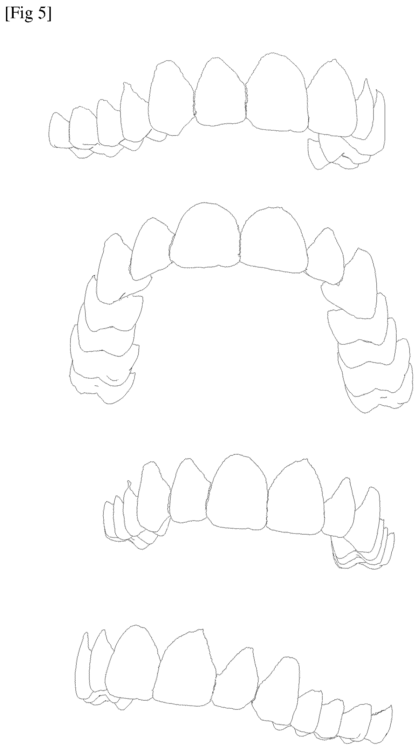

La figure 5 représente un ensemble d'images actualisées, prises selon différentes angulations, après traitement avec un réseau de neurones ainsi entrainé.

L'angulation peut être également identifiée par un réseau de neurones entrainé à cet effet. Le réseau de neurones est de préférence choisi parmi les CNN, la dernière couche du réseau de neurones opérant une régression.

Ledit réseau de neurones est entrainé avec une base d'apprentissage constituée d'un ensemble de plus de 1000, de préférence plus de 10 000 enregistrements comportant chacun :
- une image comportant une zone représentant l'organe dentaire, par exemple comportant au moins une zone de dent relative à une dent ayant un numéro déterminé ;
- un descriptif de ladite image identifiant l'angulation de ladite image.

Lors de l'entrainement, chaque image est fournie à l'entrée du réseau de neurones alors que le descriptif associé est fourni à la sortie de réseau de neurones.

A l'issue dudit entrainement, le réseau de neurones est ainsi apte à de déterminer l'angulation d'une image qu'on lui fournit en entrée.

De préférence, l'applicatif définit un ensemble d'angulations prédéterminées et, pour chaque angulation prédéterminée, un nombre d'images actualisées à acquérir. Lorsque l'applicatif est activé, à l'étape b), il met de préférence en œuvre les étapes suivantes, en temps réel :
- à un instant actuel, analyse de l'image affichée sur l'écran de l'appareil d'acquisition d'images, ou « image actuelle », de manière à déterminer si l'organe dentaire est représenté, et de préférence l'angulation de l'appareil d'acquisition d'images, ou « angulation actuelle » ;
- si l'organe dentaire est représenté et, de préférence si l'angulation actuelle est conforme au besoin actuel, c'est-à-dire s'il est encore nécessaire, à l'instant actuel, d'acquérir une image actualisée avec l'angulation actuelle, déclenchement de ladite acquisition par l'opérateur ou automatiquement ;
sinon, de préférence, information de l'opérateur pour qu'il modifie l'angulation de l'appareil d'acquisition d'images ou, s'il n'existe plus de besoin pour l'acquisition d'une image actualisée supplémentaire, pour qu'il mette fin à l'étape b).

Dans un mode de réalisation préféré, le déclenchement de l'acquisition est automatique, c'est-à-dire sans action d'un opérateur, dès que l'image affichée représente l'organe dentaire et que l'angulation est approuvée par l'appareil d'acquisition d'images.

Pour guider l'opérateur, des messages écrits et/ou vocaux peuvent être émis par l'appareil d'acquisition d'images. Par exemple, l'appareil d'acquisition d'images peut annoncer « prenez une photo de face », émettre un signal pour informer l'opérateur que l'orientation est acceptable ou qu'au contraire, il doit refaire une photo.

La fin du processus d'acquisition peut être annoncée par l'appareil d'acquisition d'images oralement ou par affichage sur l'écran.

Un guidage de l'opérateur lors de l'acquisition des images actualisées permet avantageusement de constituer un ensemble d'images actualisées immédiatement adapté au réseau de neurones entrainé.

### Constitution de l'ensemble d'images actualisées après l'acquisition

L'ensemble d'images actualisées peut être également être constitué, partiellement ou totalement, après l'acquisition des images actualisées, en sélectionnant un nombre suffisant d'images actualisées qui représentent l'organe dentaire actualisé suivant des angulations souhaitées.

Pour déterminer si une image actualisée peut appartenir à un ensemble d'images actualisées spécialisé pour un organe dentaire actualisé, par exemple un numéro de dent, on recherche si cette image actualisée représente cet organe dentaire actualisé, par exemple une dent ayant ce numéro, et, de préférence, on vérifie que l'angulation est adaptée.

La sélection peut être manuelle, en particulier lorsque les angulations souhaitées sont grossières. Il est par exemple simple de prendre une photo vue de face, une photo vue de droite bouche ouverte, une photo de gauche bouche ouverte, une photo vue de droite bouche fermée, et une photo de gauche bouche fermée.

La sélection des images actualisées peut être également faite par ordinateur. Des réseaux de neurones comme ceux décrits ci-dessus pour l'acquisition des images peuvent être utilisés. L'identification d'un organe dentaire et/ou la détermination de l'angulation peuvent être également réalisées par une analyse conventionnelle de l'image, mais une telle analyse est lente.

Si l'image actualisée représente l'organe dentaire avec une angulation et que l'ensemble d'images actualisées nécessite encore une image actualisée supplémentaire pour cette angulation, l'image actualisée est ajoutée audit ensemble.

Si l'image actualisée représente l'organe dentaire avec une angulation souhaitée, mais qu'elle est surabondante, elle peut remplacer une autre image actualisée présente dans l'ensemble spécialisé si sa qualité est supérieure, par exemple parce qu'elle représente plus de surface de l'organe dentaire que ladite autre image actualisée.

**A l'étape c),** l'ensemble des images actualisées constitué à l'étape b) est entré dans le réseau de neurones entrainé à l'étape a).

Le réseau de neurones fournit en réponse une information spatiale actualisée relative à l'organe dentaire actualisé.

### Exécution multiple avec différents réseaux de neurones

Le réseau de neurones est de préférence spécialisé pour un organe dentaire limité, par exemple une dent ayant un numéro déterminé, et l'attribut spatial comporte de préférence un nombre réduit de variables. De préférence, le procédé d'analyse est alors exécuté plusieurs fois à l'instant actualisé, en modifiant à chaque fois l'organe dentaire considéré et/ou l'attribut spatial considéré.

L'ensemble des informations spatiales actualisées ainsi déterminé est appelé « information statique ». Il permet d'obtenir une analyse détaillée de la situation dentaire du patient actualisé, en multipliant le recours à des réseaux de neurones spécialisés.

Dans un mode de réalisation préféré, à l'étape a), plusieurs réseaux de neurones spécialisés sont entrainés, chaque réseau de neurones étant spécialisé sur un organe dentaire, de préférence spécialisé pour un type de dent ou un numéro de dent respectif. De préférence, on acquiert alors à l'étape b) un nombre d'images actualisées suffisant pour constituer des ensembles d'images actualisées « spécialisés » adaptés pour chacun des réseaux de neurones spécialisés.

De préférence, les images actualisées sont toutes acquises sensiblement simultanément. Si nécessaire, le lot des images actualisées acquises est analysé afin d'identifier le ou les ensembles spécialisés auxquels elles peuvent appartenir.

L'information statique peut être enrichie. Par exemple, pour chacune d'un triplet de dents adjacentes constitué d'une dent centrale, d'une dent gauche et d'une dent droite, la dent centrale étant entre les dents gauche et droite, le procédé d'analyse peut être mis en œuvre pour déterminer la position du barycentre de la dent dans un repère fixé par rapport à l'arcade dentaire portant ces dents. L'ensemble de ces trois positions est une information statique. Pour enrichir cette information statique, on peut déterminer, à partir des trois positions, un angle formé par deux segments de droite ayant pour origine commune le barycentre de la dent centrale et passant respectivement par les barycentres des dents gauche et droite. On peut également déterminer les distances entre le barycentre de la dent centrale d'une part et le barycentre de la dent gauche ou le barycentre de la dent droite.

Le procédé d'analyse peut être exécuté plusieurs fois, en modifiant à chaque fois l'attribut spatial considéré. Par exemple, le procédé d'analyse peut être mis en œuvre pour déterminer la position du point de contact d'une dent avec une première dent adjacente, puis pour déterminer la position du point de contact de la dent avec une deuxième dent adjacente. L'ensemble des coordonnées définissant ces deux positions est une information statique.

Le procédé d'analyse est de préférence exécuté plusieurs fois, en modifiant à chaque fois l'organe dentaire considéré ou l'attribut spatial considéré. Si on considère par exemple un triplet de dents adjacentes constitué d'une dent centrale, d'une dent gauche et d'une dent droite, la dent centrale étant entre les dents gauche et droite, le procédé d'analyse peut être mis en œuvre successivement pour déterminer, dans un repère fixé par rapport à l'arcade dentaire portant ces dents, la position du barycentre de la dent gauche, puis la position du barycentre de la dent droite, puis pour déterminer la position du point de contact de la dent centrale avec la dent gauche, puis la position du point de contact de la dent centrale avec la dent droite.

Pour enrichir l'information statique ainsi obtenue, on peut par exemple mesurer un angle entre deux plans perpendiculaires à une droite reliant les deux barycentres des dents droite et gauche, et à une droite reliant les deux points de contact de la dent centrale avec les dents gauche et droite, respectivement. Cet angle fournit ainsi une information sur l'orientation de la dent centrale relativement aux dents droite et gauche.

### Utilisation de l'information statique

L'information statique peut être utilisée pour évaluer la situation dentaire du patient actualisé.

Elle peut notamment servir à évaluer si un organe dentaire actualisé est dans une position qui appartient à une région de l'espace prédéterminée, et en particulier à une région définissant un ensemble des positions prédéfinies, par exemple considérées comme acceptables. Par exemple, une telle région peut être définie autour d'une dent, ou d'un point remarquable d'une dent de manière que si la dent sort, même partiellement de cette région, ou si le point remarquable sort de cette région, la situation dentaire soit considérée comme anormale. Une telle région peut être aussi définie autour d'un appareil orthodontique, ou d'un point remarquable d'un appareil orthodontique de manière que si l'appareil orthodontique sort, même partiellement de cette région, ou si le point remarquable sort de cette région, la situation dentaire soit considérée comme anormale.

L'information statique peut servir à évaluer si un organe dentaire actualisé présente une orientation qui appartient à un ensemble d'orientations prédéterminé, et en particulier un ensemble d'orientations définissant un ensemble d'orientations considérées comme acceptables. L'orientation d'une dent peut être en particulier définie par l'angle formé par deux droites passant par un point remarquable de la dent, par exemple son barycentre, la première desdites droites passant par un point remarquable, par exemple le barycentre, d'une dent à droite de la dent, de préférence adjacente à la dent, et la deuxième desdites droites passant par un point remarquable, par exemple le barycentre, d'une dent à gauche de la dent, de préférence adjacente à la dent.

L'information statique peut encore servir à évaluer si une distance entre un point remarquable d'un organe dentaire actualisé et un autre point remarquable de l'arcade dentaire portant ledit organe dentaire actualisé, par exemple si la distance entre le barycentre d'une dent et le barycentre d'une dent adjacente à la dent, appartient à une plage de distances prédéterminée, et en particulier un ensemble de distances considérées comme acceptables.

Les limites des régions ou des plages d'acceptabilité définies pour une information statique, ou « contraintes statiques », sont de préférence définies par un professionnel de soins dentaires.

L'information statique peut être notamment utilisée pour déterminer si la situation dentaire du patient actualisé est devenue anormale. Par exemple, pour détecter la récidive, les contraintes statiques peuvent correspondre à la situation dentaire à l'issue du traitement orthodontique, avec une marge de tolérance éventuelle.

L'information statique peut être utilisée pour déterminer le positionnement d'une première arcade dentaire du patient actualisé relativement à la seconde arcade dentaire du patient actualisé, en particulier pour détecter et/ou évaluer la présence d'un surplomb vertical ou horizontal, en particulier lorsque ledit surplomb est anormal.

Pour le suivi d'un traitement orthodontique, les contraintes statiques peuvent correspondre à la situation dentaire attendue à l'instant actualisé ou à la fin du traitement orthodontique, avec une marge de tolérance éventuelle.

En dehors de tout traitement, les contraintes statiques peuvent définir un ensemble de situations dentaires considérées comme normales.

Dans un mode de réalisation, les contraintes statiques sont indépendantes du patient actualisé, c'est-à-dire applicables à tout patient d'un groupe de patients. Elles constituent ainsi un étalon, ou « set-up type ».

De préférence, l'étalon est spécifique à une pathologie et/ou à un type de traitement orthodontique, et/ou à un groupe de patients partageant une caractéristique commune, par exemple appartenant à une même classe d'âge et/ou de même sexe. L'étalon peut en particulier déterminer une forme d'arcade.

L'étalon peut en particulier définir une situation dentaire en fin d'un traitement ou à l'instant actualisé.

Un message peut être adressé au patient actualisé et/ou à un professionnel de soins dentaires pour l'informer, notamment lorsqu'une contrainte statique n'est pas respectée, par exemple si une position, une orientation et/ou une distance déterminée(s) à partir de l'information statique n'est pas (ou ne sont pas) acceptable(s).

L'information statique peut être présentée sous une forme d'un graphique.

Par exemple, l'information statique peut être présentée sur un écran d'ordinateur ou un écran de téléphone mobile.

Par exemple, le graphique peut représenter les dents avec une couleur qui dépend d'un indice de conformité exprimant la conformité de la position de chaque dent à une position prédéfinie, par exemple à une position prédéfinie à l'instant actualisé. Par exemple, plus la dent est sombre, plus sa position est écartée de la position prédéfinie.

L'information statique peut être en particulier utilisée pour
- détecter ou évaluer une position ou d'une forme d'une dent et/ou une évolution d'une position ou d'une forme d'une dent et/ou une vitesse d'évolution d'une position ou d'une forme d'une dent, notamment
   - en période de pré-traitement, c'est-à-dire dans une période qui précède un traitement orthodontique,
   - en dehors de tout traitement orthodontique, en particulier pour suivre l'éruption d'une dent ou pour détecter une récidive ou une position anormale d'une dent ou pour détecter une abrasion d'une dent, par exemple du fait d'un bruxisme, ou pour suivre l'ouverture ou la fermeture d'un espace entre deux ou plus de deux dents, notamment entre deux dents adjacentes, ou pour contrôler la stabilité ou la modification de l'occlusion,
   - dans le cadre d'un traitement orthodontique, en particulier pour suivre le déplacement d'une dent vers un positionnement prédéterminé, en particulier un positionnement amélioré de la dent, ou pour suivre l'éruption d'une dent, ou pour suivre l'ouverture ou la fermeture d'un espace entre deux ou plus de deux dents, notamment entre deux dents adjacentes, par exemple pour créer un espace adapté à la pose d'un implant dentaire, et/ou
- détecter ou évaluer une position ou une forme d'un appareil orthodontique, en particulier une position ou une forme anormale d'un appareil orthodontique, par exemple un décollement d'une bague ou d'une gouttière orthodontique, et/ou une évolution d'une position ou d'une forme d'un appareil orthodontique et/ou une vitesse d'évolution d'une position ou d'une forme d'un appareil orthodontique, notamment pour
   - optimiser la date de prise de rendez-vous chez un orthodontiste ou un dentiste, et/ou
   - évaluer l'efficacité d'un traitement orthodontique actif, et/ou
   - mesurer l'activité d'un appareil orthodontique actif ; et/ou
   - mesurer une perte d'efficacité d'un appareil orthodontique passif ; et/ou
- en dentisterie, et/ou
- mesurer une évolution de la forme de la denture du patient entre deux dates, par exemple en pré-traitement, en particulier entre deux dates séparées par un évènement susceptible d'avoir modifié la position et/ou la forme d'au moins une dent, par exemple séparées par l'occurrence d'un choc sur les dents ou par la mise en œuvre d'un dispositif dentaire susceptible de produire un effet indésirable, par exemple destiné au traitement de l'apnée du sommeil, ou par l'occurrence d'une greffe dans la bouche du patient, notamment d'une greffe en parodontie, et en particulier d'une greffe de gencive.

Lorsque l'information statique est utilisée pour évaluer une évolution, elle peut être comparée à une situation définie, à un instant antérieur à l'instant actualisé, sans avoir eu recours à un procédé selon l'invention. Par exemple, lorsque l'information statique est utilisée pour évaluer une évolution d'une position ou d'une forme d'une dent, elle peut être comparée à une position ou une forme de cette dent prédéfinie sans mettre en œuvre les étapes a) à c), ladite position ou forme étant par exemple prédéfinie au début du traitement ou à un instant intermédiaire du traitement.

### Exécution multiple à différents instants actualisés : quantités de mouvement

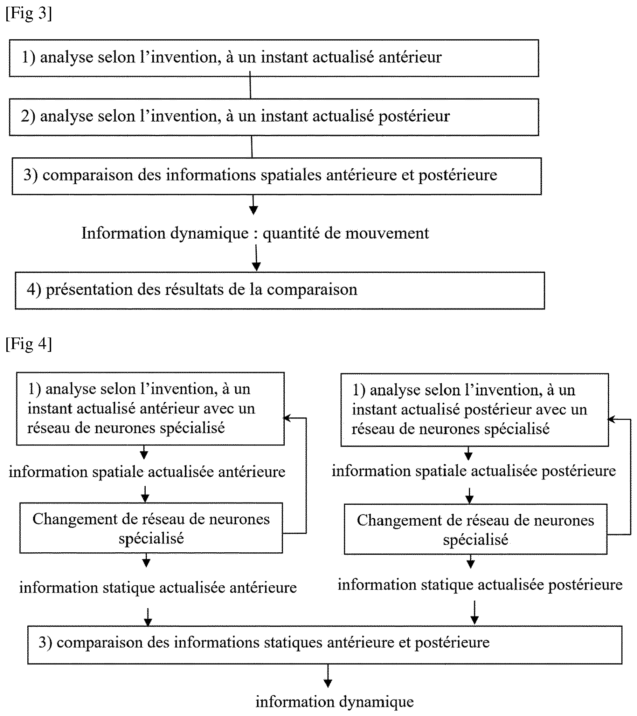

Un procédé d'analyse selon l'invention peut être également mis en œuvre, une ou plusieurs fois, à des instants actualisés « antérieur » et « postérieur » différents, comme illustré sur la figure 3.

La comparaison de l'information spatiale actualisée (ou de l'information statique) « antérieure » obtenue à l'instant actualisé antérieur avec l'information spatiale actualisée (ou avec l'information statique, respectivement) « postérieure » obtenue à l'instant actualisé postérieur permet de déterminer une évolution entre ces deux instants actualisés. Cette évolution, ramenée à l'intervalle temporel entre ces deux instants actualisés, permet de déterminer une vitesse de cette évolution.

On appelle « quantité de mouvement » l'information résultant directement ou indirectement d'une telle comparaison.

La quantité de mouvement peut être en particulier un déplacement d'un point remarquable entre les deux instants actualisés ou, par division de ce déplacement par l'intervalle de temps entre ces deux instants actualisés, une vitesse moyenne de déplacement entre ces instants actualisés.

L'invention concerne ainsi un procédé de détermination d'une quantité de mouvement d'un organe dentaire d'un patient actualisé comportant les étapes 1) à 3), et optionnellement 4).

**A l'étape 1),** on met en œuvre un procédé d'analyse selon l'invention, à l'instant actualisé antérieur, de manière à obtenir l'information spatiale antérieure relative à l'organe dentaire actualisé.

L'instant actualisé antérieur peut être par exemple moins de 3 mois, moins de 2 mois, moins de 1 mois, moins d'une semaine, moins de 2 jours après la pose d'un appareil orthodontique actif ou passif, par exemple d'une gouttière orthodontique, d'un arc orthodontique ou d'une attelle de contention.

Le procédé d'analyse selon l'invention peut être mis en œuvre plusieurs fois, comme décrit précédemment, en fonction de l'information statique antérieure souhaitée.

**A l'étape 2),** on met en œuvre le même procédé d'analyse selon l'invention, à l'instant actualisé postérieur, de manière à obtenir l'information spatiale postérieure. L'information spatiale postérieure est donc relative au même organe dentaire actualisé et au même attribut spatial que le procédé d'analyse mis en œuvre à l'instant antérieur.

L'instant actualisé postérieur est de préférence postérieur à l'instant actualisé antérieur de plus de 2 semaines, 1 mois, 2 mois ou 6 mois et/ou de moins de 5 ans, 3 ans ou 1 an.

A l'étape 2), on utilise le ou les mêmes réseaux de neurones qu'à l'étape 1). L'étape a) n'est donc pas nécessaire.

Si le procédé d'analyse selon l'invention a été mis en œuvre plusieurs fois à l'étape 1), il en est de même à l'étape 2), de manière à obtenir une information statique postérieure comparable à l'information statique antérieure.

**A l'étape 3),** on compare les informations spatiales antérieure et postérieure pour déterminer une quantité de mouvement.

Les informations spatiales antérieure et postérieure sont des valeurs qui peuvent être comparées les unes aux autres, par exemple par différence.

Par exemple, si les informations spatiales antérieure et postérieure sont constituées des coordonnées (x₁, y₁ et z₁) et (x₂, y₂ et z₂) du barycentre d'une dent, aux instants actualisés antérieur t₁ et postérieur t₂, respectivement, dans un repère fixe par rapport à l'arcade dentaire, la racine carrée de (x₂-x₁)² + (y₂-y₁)² + (z₂-z₁)² permet d'évaluer la distance parcourue par ce barycentre entre les instants actualisés antérieur t₁ et postérieur t₂.

Le résultat de la comparaison peut être constitué d'une ou de plusieurs valeurs. Par exemple, considérons la situation dans laquelle l'attribut spatial est (x' ; y' ; z' ; x" ; y" ; z"), (x' ; y' ; z') et (x" ; y" ; z") étant les positions de deux points remarquables P' et P", respectivement, d'une dent dans un repère tridimensionnel, par exemple orthonormé, (Ox ; Oy ; Oz), l'origine étant par exemple au centre de l'arcade dentaire portant cette dent. Si on note l'information spatiale antérieure et postérieure (x₁^{'} ; y₁^{'} ; z₁^{'} ; x₁" ; y₁" ; z₁") et (x₂^{'} ; y₂^{'} ; z₂^{'} ; x₂" ; y₂" ; z₂"), respectivement,
- x₁^{'}, y₁^{'}, z₁^{'}, sont les valeurs des coordonnées x, y et z, par exemple en mm, du point P' à l'instant actualisé antérieur,
- x₂^{'}, y₂^{'}, z₂^{'} sont les valeurs des coordonnées x, y et z, par exemple en mm, du point P' à l'instant actualisé postérieur,
- x₁", y₁",z₁" sont les valeurs des coordonnées x, y et z, par exemple en mm, du point P" à l'instant actualisé antérieur, et
- x₂", y₂", z₂" sont les valeurs des coordonnées x, y et z, par exemple en mm, du point P" à l'instant actualisé postérieur.

Le résultat de la comparaison peut être alors constitué des distances parcourues, entre les instants actualisés antérieur t₁ et postérieur t₂, par le point P' et par le point P", par exemple comme déterminé ci-dessus (deux valeurs pour le résultat de la comparaison), ou être la moyenne arithmétique de ces deux distances (une valeur pour le résultat de la comparaison).

**A l'étape 4),** de préférence, on présente ladite quantité de mouvement, par exemple sur un écran d'ordinateur personnel ou d'un téléphone mobile du patient actualisé et/ou d'un professionnel de soins dentaires.

Comme illustré sur la figure 4, les étapes 1) et 2) peuvent être mises en œuvre plusieurs fois, en modifiant à chaque fois l'organe dentaire actualisé et/ou l'attribut spatial du procédé d'analyse. Par exemple, elles peuvent être répétées pour chacune d'une pluralité de dents du patient actualisé. A l'étape 3), on peut comparer les informations spatiales antérieure et postérieure actualisées obtenues pour chaque couple d'étapes 1) et 2). Les informations spatiales antérieure et postérieure actualisées obtenues pour différents couples d'étapes 1) et 2) peuvent être également combinées pour obtenir une information enrichie.

On appelle globalement « information dynamique » l'ensemble des informations résultant directement ou indirectement de la mise en œuvre, une fois ou plusieurs fois, des étapes 1) à 3), à chaque fois à l'instant actualisé antérieur t₁ et à l'instant actualisé postérieur t₂.

### Utilisation de l'information dynamique

L'information dynamique peut être utilisée pour évaluer une évolution de la situation dentaire du patient actualisé.

Elle peut servir à évaluer si une vitesse de déplacement, en translation ou en rotation, d'un point remarquable de l'organe dentaire actualisé est dans une plage de valeurs définissant un ensemble des vitesses prédéfini, par exemple un ensemble de vitesses considérées comme acceptables.

L'information dynamique peut servir à évaluer si la dynamique d'un traitement orthodontique actif est conforme à ce qui avait été anticipé, c'est-à-dire si les dents bougent à une vitesse qui est conforme au traitement orthodontique.

Les limites des plages d'acceptabilité définies pour une information dynamique, ou « contraintes dynamiques », sont de préférence définies par un professionnel de soins dentaires.

L'information dynamique peut être présentée sous une forme d'un graphique.

Par exemple, l'information dynamique peut être présentée sur un écran d'ordinateur ou un écran de téléphone mobile.

De préférence, le graphique synthétise l'ensemble des informations résultant directement ou indirectement de la mise en œuvre, une fois ou plusieurs fois, des étapes 1) à 3), à chaque fois à l'instant actualisé antérieur t₁ et à l'instant actualisé postérieur t₂.

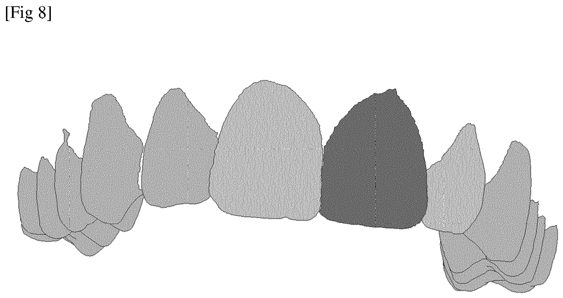

Par exemple, sur la figure 8, les dents seront colorées en fonction d'un indice de conformité qui exprime la conformité de la vitesse de déplacement de chaque dent à une vitesse prédéfinie, par exemple dans le cadre d'un traitement orthodontique subi par le patient actualisé.

L'exploitation de l'information dynamique est généralement plus simple que celle de l'information statique. Par exemple, il est généralement plus facile de déterminer si un point remarquable d'une dent s'est déplacé de manière anormale que de déterminer si une position de ce point dans l'espace est anormale.

Par exemple, pour détecter la récidive, les contraintes dynamiques peuvent correspondre à une marge de déplacement autorisée pour le barycentre d'une dent. Pour le suivi d'un traitement orthodontique, les contraintes dynamiques peuvent correspondre à un sens de déplacement d'une dent par rapport à une autre (réduction ou augmentation de la distance entre ces dents), pour vérifier que les deux dents se rapprochent ou s'écartent l'une de l'autre. Les contraintes dynamiques peuvent encore correspondre à une valeur de seuil pour une vitesse de déplacement d'un appareil orthodontique ou d'un point d'une dent sur laquelle l'appareil orthodontique agit, pour vérifier l'activité de l'appareil orthodontique.

L'information dynamique peut être également utilisée pour mesurer une évolution de la forme d'une dent ou d'un ensemble de dents.

L'information dynamique peut être ainsi en particulier utilisée pour
- détecter ou évaluer une évolution d'une position ou d'une forme d'une dent et/ou une vitesse d'évolution d'une position ou d'une forme d'une dent, notamment
   - en période de pré-traitement, c'est-à-dire dans une période qui précède un traitement orthodontique,
   - en dehors de tout traitement orthodontique, en particulier pour suivre l'éruption d'une dent ou pour détecter une récidive ou une position anormale d'une dent ou pour détecter une abrasion d'une dent, par exemple du fait d'un bruxisme, ou pour suivre l'ouverture ou la fermeture d'un espace entre deux ou plus de deux dents, notamment entre deux dents adjacentes, ou pour contrôler la stabilité ou la modification de l'occlusion,
   - dans le cadre d'un traitement orthodontique, en particulier pour suivre le déplacement d'une dent vers un positionnement prédéterminé, en particulier un positionnement amélioré de la dent, ou pour suivre l'éruption d'une dent, ou pour suivre l'ouverture ou la fermeture d'un espace entre deux ou plus de deux dents, notamment entre deux dents adjacentes, par exemple pour créer un espace adapté à la pose d'un implant dentaire, et/ou
- détecter ou évaluer une évolution d'une position ou d'une forme d'un appareil orthodontique et/ou une vitesse d'évolution d'une position ou d'une forme d'un appareil orthodontique, en particulier une position ou une forme anormale d'un appareil orthodontique, par exemple un décollement d'une bague ou d'une gouttière orthodontique, notamment pour
   - optimiser la date de prise de rendez-vous chez un orthodontiste ou un dentiste, et/ou
   - évaluer l'efficacité d'un traitement orthodontique actif, et/ou
   - mesurer l'activité d'un appareil orthodontique actif ; et/ou
   - mesurer une perte d'efficacité d'un appareil orthodontique passif ; et/ou
- en dentisterie, et/ou
- mesurer une évolution de la forme de la denture du patient entre deux dates, par exemple en pré-traitement, en particulier entre deux dates séparées par un évènement susceptible d'avoir modifié la position et/ou la forme d'au moins une dent, par exemple séparées par l'occurrence d'un choc sur les dents ou par la mise en œuvre d'un dispositif dentaire susceptible de produire un effet indésirable, par exemple destiné au traitement de l'apnée du sommeil, ou par l'occurrence d'une greffe dans la bouche du patient, notamment d'une greffe en parodontie, et en particulier d'une greffe de gencive.

Un message peut être adressée au patient actualisé et/ou à un professionnel de soins dentaires pour l'informer, notamment lorsqu'une contrainte dynamique n'est pas respectée, par exemple si une amplitude et/ou une vitesse et/ou une direction d'un déplacement d'un point remarquable d'une dent déterminée(s) à partir de l'information dynamique n'est pas (ou ne sont pas) acceptable(s).

Dans un mode de réalisation, l'information statique et/ou l'information dynamique sont utilisées pour évaluer si un objectif est atteint et/ou pour mesurer la différence entre la situation dentaire du patient actualisé à l'instant actualisé et la réalisation de l'objectif.

L'objectif est de préférence choisi parmi les objectifs suivants :
- le patient actualisé atteint une classe d'occlusion n°I pour les canines,
- le patient actualisé atteint une classe d'occlusion n°I pour les molaires,
- les espaces du secteur antérieur du patient actualisé sont fermés,
- l'espace résultant de l'extraction d'une dent du patient actualisé est fermé,
- le patient actualisé présente un surplomb horizontal , ou « overjet » en anglais, normal, de préférence compris entre 1 et 3 mm,
- le patient actualisé présente un surplomb vertical, ou « overbite » en anglais, normal, de préférence compris entre 1 et 3 mm,
- les milieux inter-incisifs des arcades supérieure et inférieure du patient actualisé ne sont pas décalés,
- le patient actualisé ne présente pas de décalage latéral de l'arcade inférieure et/ou de l'arcade supérieure par rapport à un plan sagittal de la tête du patient, ,
- le patient actualisé ne présente pas de décalage latéral de l'arcade supérieure par rapport à l'arcade inférieure,
- un appareil orthodontique, par exemple l'arc orthodontique et/ou la gouttière orthodontique et/ou les appareils auxiliaires, porté par le patient actualisé est passif, c'est-à-dire n'agit plus pour modifier les positions des dents du patient actualisé ;
- pas ou peu de mouvements de dents du patient actualisé détectés lors du ou des deux derniers contrôles de l'arcade supérieure et/ou inférieure, de préférence aucun mouvement d'une dent du patient actualisé n'a été détecté,
- toutes les dent temporaires du patient actualisé sont tombées,
- absence de béance latérale (fermeture de la béance latérale),
- absence de béance postérieure,
- absence de béance antérieure,
- absence d'inversée d'articulé antérieur,
- absence d'inversée d'articulé postérieur,
- amélioration de l'encombrement,
- stabilisation des récessions,
- diastèmes fermés,
- absence d'irrégularités de la muqueuse.

### Exemple

Dans un exemple, l'organe dentaire considéré est un couple de deux dents d'un type ou d'un numéro déterminé, par exemple la dent n°13 (canine supérieure droite) et la dent adjacente n°14 (première prémolaire supérieure droite). L'attribut spatial est un triplet de coordonnées, ou « paramètres », (X, Y, Z) pour un vecteur joignant le barycentre de la dent n°13 et le barycentre de la dent n°14. Une information spatiale est donc constituée d'un triplet de valeurs pour ces coordonnées.

L'information spatiale est mesurée par rapport à un repère orthonormé (Ox ; Oy ; Oz) dont l'origine O est au centre de l'arcade dentaire supérieure du patient considéré.

A l'étape A), on crée une base d'apprentissage historique comportant 100 000 enregistrements historiques.

Les images historiques sont toutes des photos extraorales, en couleurs réelles, prises sans écarteur, puis croppées.

Les photos ont le plus souvent été prises avec un appareil photo personnel, généralement un téléphone mobile, et parfois avec un appareil photo d'un professionnel de soins dentaires. Elles ont été prises à des distances variables du patient historique, puis, de préférence croppées de manière que la taille des dents n°13 et 14 soit sensiblement la même quelle que soit l'image historique considérée.

Tout enregistrement historique comporte un ensemble de quatre images historiques d'un patient historique qui représentent toutes les dents n°13 et n°14 du patient historique, et dont les angulations sont respectivement « vue de face », « vue de dessous », « vue de droite » et « vue de face-droite » (dans le plan occlusal).

L'information spatiale historique d'un enregistrement historique est constituée d'un vecteur (Xi, Yi, Zi). Autrement dit, depuis le barycentre de la dent n°13, un déplacement d'une valeur Xi selon l'axe Ox, puis d'une valeur Yi selon l'axe Oy, puis d'une valeur Zi selon l'axe Oz conduit au barycentre de la dent n°14. L'information spatiale historique de chaque enregistrement est déterminée manuellement, à partir d'un modèle tridimensionnel des arcades dentaires du patient historique réalisé avec un scanner 3D.

A l'étape B), on entraine un réseau de neurones CNN, par exemple GoogleNet (2015), avec la base d'apprentissage historique de manière qu'il soit capable de déterminer un vecteur entre les barycentres des dents n°13 et n°14 à partir d'un ensemble d'images actualisées similaire aux ensembles d'images historiques utilisés pour l'entrainement.

A l'étape b), on considère un patient actualisé, par exemple une personne qui ne prévoit aucun traitement orthodontique et ne porte pas d'attelle de contention, à un instant actualisé antérieur t₁. Elle dispose d'un téléphone mobile dans lequel elle a chargé un applicatif capable de mettre en œuvre les étapes b) et c). Elle souhaite vérifier la situation dentaire relative à ses dents n°13 et n°14 et lance cet applicatif.

L'applicatif active l'appareil photo du téléphone mobile et guide le patient actualisé pour qu'il acquiert quatre photos représentant ces deux dents suivantes lesdites angulations « vue de face », « vue de dessous », « vue de droite » et « vue de face-droite ».

L'applicatif croppe alors ces photos, prises à des distances variables du patient actualisé, de manière que la taille des dents n°13 et 14 soit sensiblement la même quelle que soit l'image actualisée, et sensiblement identique à celle de ces dents sur les images historiques.

L'applicatif soumet alors l'ensemble des quatre images actualisées au réseau de neurones entrainé. Ce dernier peut être intégré dans l'applicatif ou être sur un ordinateur à distance du téléphone mobile, auquel cas le téléphone mobile transmet les quatre images actualisées à l'ordinateur à distance pour qu'il les soumette à l'entrée du réseau de neurones entrainé.

A l'étape c), à partir de ces seules quatre images actualisées, le réseau de neurones entrainé fournit, de préférence en moins de 120 s, 60 s, 40s, 20 s, 10 s ou 5 s, une information spatiale actualisée. L'information spatiale actualisée est un vecteur (Xa, Ya, Za) qui dans le repère orthonormé (Ox ; Oy ; Oz) dont l'origine O est au centre de l'arcade dentaire supérieure du patient actualisé, permet de relier le barycentre de la dent n°13 du patient actualisé au barycentre de sa dent n°14.

Le vecteur (Xa, Ya, Za) est une information statique qui peut être comparée à des contraintes statiques prédéfinies. Par exemple, on peut vérifier si |Xa| < Sx, | et/ou si |Ya| < Sy, et/ou si |Za| < Sz, Sx, Sy et Sz étant des valeurs de seuil, par exemple de 0,5 mm, 0,7 mm et 0,3 mm. On peut aussi vérifier, par exemple, si |Xa| + |Ya| + |Za| < S, S étant une valeur de seuil. Si une contrainte n'est pas respectée, par exemple parce que |Xa| > Sx, un message est envoyé au patient actualisé pour l'avertir. Par exemple, un message est affiché sur son écran de téléphone mobile pour l'inviter à contacter un professionnel de soins dentaires.

Si le réseau de neurones est dans un ordinateur à distance, ce dernier transmet à l'applicatif l'information spatiale actualisée et/ou ledit message.

Le patient actualisé peut effectuer les mêmes démarches (activation de l'applicatif, prise de photos et soumission au réseau de neurones entrainé) à un instant actualisé postérieur t₂, par exemple un mois après l'instant actualisé antérieur.

Pour cette deuxième mise en œuvre de l'invention, l'applicatif peut non seulement analyser l'information statique obtenue à l'instant actualisé postérieur, à savoir un vecteur (Xa', Ya', Za'), comme à l'instant actualisé antérieur, mais aussi la comparer à l'information statique obtenue à l'instant actualisé antérieur, c'est-à-dire au vecteur (Xa, Ya, Za). Il peut par exemple déterminer les quantités de mouvement suivantes : |Xa' - Xa|, |Ya' - Ya|, |Za' - Za|, |Xa' - Xa| + |Ya' - Ya| + |Za' - Za|, |Xa' - Xa|/ (t₂-t₁), |Ya' - Ya|/ (t₂-t₁), |Za' - Za|/ (t₂-t₁), ou (|Xa' - Xa| + |Ya' - Ya| + |Za' - Za|)/ (t₂-t₁).

Ces quantités de mouvement constituent une information dynamique qui renseigne avantageusement sur l'évolution dans le temps de la situation dentaire relative aux dents n°13 et 14, et plus précisément sur le déplacement relatif de la dent n°13 par rapport à la dent n°14. Elles complètent donc l'information statique.

L'information dynamique peut être comparée à des contraintes dynamiques prédéfinies. Par exemple, on peut vérifier si |Xa' - Xa|/ (t₂-t₁) < Vx, |Ya' - Ya|/ (t₂-t₁) < Vy, |Za' - Za|/ (t₂-t₁) < Vz, ou si (|Xa' - Xa| + |Ya' - Ya| + |Za' - Za|)/ (t₂-t₁) < V, Vx, Vy, Vz et V étant des valeurs de seuil, par exemple de 0,1 mm/mois, 0,2 mm/mois 0,1 mm/mois et 0,3 mm/mois, respectivement. Si une contrainte n'est pas respectée, un message est envoyé au patient actualisé pour l'avertir. Par exemple, un message est affiché sur son écran de téléphone mobile pour l'inviter à contacter un professionnel de soins dentaires.

Si le réseau de neurones est dans un ordinateur à distance, ce dernier transmet à l'applicatif l'information spatiale actualisée et/ou tout ou partie de l'information dynamique et/ou ledit message.

De préférence, le patient actualisé met en œuvre les opérations décrites ci-dessus pour l'ensemble des couples de dents de ses arcades dentaires (dents n°1 et n°2, dents n°2 et n°3, etc.). Pour chaque couple de dent, l'applicatif met de préférence en œuvre une procédure spécialisée pour disposer de suffisamment de photos prises selon les différentes angulations prédéfinies pour le couple de dents considéré. Les photos pour un couple de dent déterminé sont soumises à un réseau de neurones spécialisé pour ce couple de dent.

Dans un mode de réalisation, l'information dynamique est utilisée pour mesurer l'efficacité, ou « activité », d'un appareil orthodontique actif, c'est-à-dire sa capacité à agir sur l'arcade dentaire à l'instant actualisé. Par exemple, les vitesses de déplacement des dents permettent de déterminer si l'appareil orthodontique continue à être efficace (si ces vitesses sont supérieures à des valeurs de seuil, par exemple à 0,1 mm/mois), et donc de déterminer si l'appareil orthodontique doit être changé ou modifié et/ou si un rendez-vous doit être pris avec un professionnel de soins dentaires. Le cas échant, un message, écrit ou oral, est adressé au patient actualisé et/ou au professionnel de soins dentaires.

### Exemple

La figure 6 illustre un exemple de mise en œuvre d'un procédé selon l'invention. Les dates sont en abscisse. L'axe des ordonnées fournit le cumul des déplacements dans toutes les directions, en millimètres, pour toutes les dents de l'arcade mandibulaire d'un patient.

La courbe en trait plein représente l'évolution « réelle ». Pour déterminer cette courbe, un modèle tridimensionnel numérique de l'arcade dentaire du patient est initialement généré avec un scanner 3D. Il est ensuite déformé pour correspondre à l'agencement des dents observé à différents instants, en mettant en œuvre le procédé décrit dans PCT/EP2015/074859. Chaque point de cette courbe nécessite plusieurs heures de traitement informatique.

La courbe en trait interrompu représente l'évolution déterminée suivant l'invention. Chaque point de cette courbe ne nécessite que quelques secondes de traitement informatique.

De manière très surprenante, on constate que la courbe en trait interrompu suit remarquablement bien la courbe en trait plein. Elle représente donc l'évolution réelle de façon réaliste, bien qu'elle soit très rapide à calculer.

Comme cela apparaît clairement à présent, l'invention fournit une solution pour déterminer des positions, des distances, des directions d'orientation ou des sens d'orientation dans le volume de la cavité buccale d'un patient. Cette solution est rapide, fiable et ne requiert que des ressources informatiques limitées.

Elle peut être en particulier mise en œuvre en quelques secondes, avec un applicatif chargé dans un téléphone mobile.

En outre, elle fournit des informations précises, la précision étant typiquement inférieure à 0,3 mm.

Enfin, elle peut être mise en œuvre à partir de simples photos extra-orales, prises par le patient lui-même avec son téléphone mobile, sans précautions particulières et sans qu'aucun modèle 3D ait besoin d'avoir été généré préalablement.

L'invention permet ainsi d'évaluer la situation dentaire d'une personne quelconque, lors d'un traitement orthodontique actif ou passif, mais aussi en dehors de tout traitement orthodontique, sans même que cette personne ait préalablement rencontré un professionnel de soins dentaires.

## Revendications

1. Procédé mis en œuvre par ordinateur, d'entrainement d'un réseau de neurones destiné à l'analyse d'une situation dentaire d'un patient actualisé, ledit procédé comportant les étapes suivantes :
A) création d'une base d'apprentissage historique relative à un organe dentaire et à un attribut spatial associé à l'organe dentaire,
la base d'apprentissage historique comportant plus de 1 000 enregistrements historiques, chaque enregistrement historique, relatif à un patient historique respectif, comportant :
- un ensemble d'images historiques représentant toutes ledit organe dentaire chez ledit patient historique, dit « organe dentaire historique » ; et
- une information spatiale comportant, pour le patient historique, un ensemble de valeurs pour ledit attribut spatial, dite « information spatiale historique » ;
B) entraînement du réseau de neurones, en lui fournissant en entrée lesdits ensembles d'images historiques et en sortie lesdites informations spatiales historiques, l'attribut spatial définissant une suite ordonnée de variables dans un repère tridimensionnel, l'attribut spatial définissant:
- une position d'un ou plusieurs points remarquables de l'organe dentaire dans un repère tridimensionnel ; et/ou
- un ou plusieurs vecteurs entre des points remarquables de l'organe dentaire et/ou entre un point remarquable de l'organe dentaire et un autre point.

2. Procédé d'entrainement selon la revendication immédiatement précédente, dans lequel le réseau de neurones est convolutif.

3. Procédé d'entrainement selon l'une quelconque des revendications précédentes, dans lequel
- l'organe dentaire est une dent ou un ensemble de moins de 5 dents ; et/ou
- l'attribut spatial comporte moins de 30 variables ; et/ou
- l'ensemble d'images historiques de tout enregistrement historique comporte plus de deux et moins de 30 images historiques ; et/ou
- chaque image historique de l'ensemble d'images historiques de tout enregistrement historique présente une angulation choisie dans un groupe d'angulations potentielles comportant plus de 2 et moins de 30 angulations potentielles, et/ou
- au moins trois images historiques de tout ensemble d'images historiques présentent des angulations différentes.

4. Procédé d'entrainement selon l'une quelconque des revendications précédentes, dans lequel l'organe dentaire historique est une dent ayant un numéro prédéterminé ou un ensemble de dents comportant une dent ayant un numéro prédéterminé et une ou deux dents adjacentes à ladite dent.

5. Procédé d'entrainement selon l'une quelconque des revendications précédentes, dans lequel les images historiques sont des photographies en couleurs réelles, et/ou ne représentent pas un écarteur dentaire, et/ou sont extra-orales.

6. Procédé implémenté par ordinateur, d'analyse d'une situation dentaire d'un patient dit « patient actualisé », à un instant actualisé, ledit procédé comportant les étapes suivantes :
a) avant l'instant actualisé, entrainement d'un réseau de neurones suivant un procédé d'entrainement selon l'une quelconque des revendications précédentes ;
b) à l'instant actualisé, acquisition, au moyen d'un appareil d'acquisition d'images, d'un ensemble d'images actualisées compatible avec ledit réseau de neurones et représentant ledit organe dentaire du patient actualisé, dit « organe dentaire actualisé » ;
c) analyse de l'ensemble d'images actualisées au moyen dudit réseau de neurones de manière à obtenir une information spatiale comportant, pour le patient actualisé, un ensemble de valeurs pour ledit attribut spatial, dite « information spatiale actualisée ».

7. Procédé d'analyse selon la revendication immédiatement précédente, dans lequel les images actualisées sont des photographies en couleurs réelles, et/ou extra-orales et/ou dans lequel, à l'étape b), l'appareil d'acquisition d'images est un téléphone mobile, et/ou le patient actualisé ne porte pas d'écarteur dentaire.

8. Procédé d'analyse selon l'une quelconque des deux revendications immédiatement précédentes, dans lequel, à l'étape b), le patient actualisé porte un écarteur dentaire.

9. Procédé d'analyse selon l'une quelconque des trois revendications immédiatement précédentes, dans lequel
- à l'étape a), on entraine une pluralité de réseaux de neurones avec des bases d'apprentissage historiques respectives qui différent en ce qu'elles concernent des organes dentaires différents et/ou des attributs spatiaux différents, de manière à obtenir une pluralité de réseaux de neurones spécialisés ;
- à l'étape b), on acquiert des images actualisées et on génère, à partir desdites images actualisées, un ensemble d'images actualisées pour chacun desdits réseaux de neurones spécialisés ;
- à l'étape c), on analyse chaque dit ensemble d'images actualisées au moyen du réseau de neurones spécialisé correspondant, de manière à obtenir une pluralité d'informations spatiales actualisées.

10. Procédé d'analyse selon la revendication immédiatement précédente, dans lequel, à l'étape a), chaque base d'apprentissage historique concerne une dent ayant un numéro spécifique à ladite base d'apprentissage historique, ou un groupe de dents, les numéros des dents dudit groupe étant spécifiques à ladite base d'apprentissage historique.

11. Procédé d'analyse selon l'une quelconque des cinq revendications immédiatement précédentes, dans lequel on utilise l'information spatiale actualisée pour évaluer si un objectif est atteint et/ou pour mesurer la différence entre la situation dentaire du patient actualisé à l'instant actualisé et la réalisation de l'objectif, l'objectif étant choisi parmi les objectifs suivants :
- le patient actualisé atteint une classe d'occlusion n°I pour les canines,
- le patient actualisé atteint une classe d'occlusion n°I pour les molaires,
- les espaces du secteur antérieur du patient actualisé sont fermés,
- l'espace résultant de l'extraction d'une dent du patient actualisé est fermé,
- le patient actualisé présente un surplomb horizontal normal,
- le patient actualisé présente un surplomb vertical normal,
- les milieux inter-incisifs des arcades supérieure et inférieure du patient actualisé ne sont pas décalés,
- le patient actualisé ne présente pas de décalage latéral de l'arcade inférieure et/ou de l'arcade supérieure par rapport à un plan sagittal de la tête du patient,
- le patient actualisé ne présente pas de décalage latéral de l'arcade supérieure par rapport à l'arcade inférieure,
- un appareil orthodontique porté par le patient actualisé n'agit plus pour modifier les positions des dents du patient actualisé,
- pas ou peu de mouvements de dents du patient actualisé détectés lors du ou des deux derniers contrôles de l'arcade supérieure et/ou inférieure,
- toutes les dent temporaires du patient actualisé sont tombées,
- absence de béance latérale,
- absence de béance postérieure,
- absence de béance antérieure,
- absence d'inversée d'articulé antérieur,
- absence d'inversée d'articulé postérieur,
- amélioration de l'encombrement,
- stabilisation des récessions,
- diastèmes fermés,
- absence d'irrégularités de la muqueuse.

12. Programme d'ordinateur comprenant des instructions de codes pour mettre en œuvre les étapes d'un procédé de détermination d'une quantité de mouvement entre un instant actualisé « antérieur » et un instant actualisé « postérieur » postérieur à l'instant actualisé antérieur, ledit procédé comportant les étapes suivantes :
1) mise en œuvre d'un procédé d'analyse selon l'une quelconque des revendications 6 à 11, à l'instant actualisé antérieur, de manière à obtenir une information spatiale actualisée antérieure ;
2) mise en œuvre d'un procédé d'analyse selon l'une quelconque des revendications 6 à 11, à l'instant actualisé postérieur, de manière à obtenir une information spatiale actualisée postérieure ;
3) comparaison des informations spatiales actualisées antérieure et postérieure, de manière à obtenir une quantité de mouvement entre les instants actualisés antérieur et postérieur ;
4) optionnellement, présentation de ladite quantité de mouvement au patient actualisé et/ou à un professionnel de soins dentaires.

13. Programme d'ordinateur selon la revendication immédiatement précédente, dans lequel la quantité de mouvement définit une amplitude et/ou une vitesse du déplacement, en translation et/ou en rotation, entre les instants actualisés antérieur et postérieur, d'un ou plusieurs points de l'organe dentaire actualisé et/ou d'un ou plusieurs vecteurs reliant des points de l'organe dentaire actualisé ou reliant un ou plusieurs points de l'organe dentaire actualisé et un ou plusieurs autres points de la cavité buccale du patient actualisé.

14. Programme d'ordinateur selon l'une quelconque des deux revendications immédiatement précédentes, dans lequel à l'étape 3), ladite quantité de mouvement est comparée à une valeur de seuil et, en fonction de la différence entre la quantité de mouvement et la valeur de seuil,
- un indice d'activité d'un appareil orthodontique porté par le patient actualisé et/ou
- un indice de conformité de la situation dentaire du patient actualisé à une situation prédéfinie par un traitement orthodontique subi par le patient actualisé, ou à une situation résultant d'un traitement orthodontique subi par le patient actualisé, ou, indépendamment d'un traitement orthodontique, à une situation définie par un professionnel de soins dentaires est déterminé.

15. Programme d'ordinateur selon l'une quelconque des trois revendications immédiatement précédentes, dans lequel l'indice d'activité et/ou l'indice de conformité sont présentés sous forme graphique.

16. Utilisation d'un procédé d'analyse selon l'une quelconque des revendications 6 à 11 pour :
- détecter ou évaluer une position ou d'une forme d'une dent, et/ou
- détecter ou évaluer une position ou une forme d'un appareil orthodontique, et/ou
- en dentisterie.

17. Utilisation selon la revendication immédiatement précédente, pour
- détecter une récidive ou une position anormale d'une dent, et/ou
- détecter une abrasion d'une dent, et/ou
- contrôler la stabilité ou la modification de l'occlusion,
- détecter ou évaluer un décollement d'une bague ou d'une gouttière orthodontique,
- optimiser la date de prise de rendez-vous chez un orthodontiste ou un dentiste, et/ou
- évaluer l'efficacité d'un traitement orthodontique actif, et/ou
- mesurer l'activité d'un appareil orthodontique actif ; et/ou
- mesurer une perte d'efficacité d'un appareil orthodontique passif.

## Patentansprüche

1. Computerimplementiertes Verfahren zum Trainieren eines neuronalen Netzes, das für die Analyse einer Zahnsituation eines aktualisierten Patienten ausgelegt ist, wobei das Verfahren die folgenden Schritte aufweist:
A) Erstellen einer historischen Lernbasis in Bezug auf ein Zahnorgan und auf ein räumliches Attribut, das mit dem Zahnorgan verknüpft ist, wobei die historische Lernbasis mehr als 1.000 historische Datensätze aufweist, wobei sich jeder historische Datensatz auf einen jeweiligen historischen Patienten bezieht und aufweist:
- einen Satz historischer Bilder, die alle das Zahnorgan des historischen Patienten darstellen, genannt "historisches Zahnorgan"; und
- eine räumliche Information, die für den historischen Patienten einen Satz von Werten für das räumliche Attribut aufweist, genannt "historische räumliche Information";
B) Trainieren des neuronalen Netzes, wobei ihm die Sätze historischer Bilder als Eingabe und die historischen räumlichen Informationen als Ausgabe bereitgestellt werden, wobei das räumliche Attribut eine geordnete Folge von Variablen in einem dreidimensionalen Referenzrahmen definiert, wobei das räumliche Attribut definiert:
- eine Position eines oder mehrerer bemerkenswerter Punkte des Zahnorgans in einem dreidimensionalen Referenzrahmen; und/oder
- ein oder mehrere Vektoren zwischen den bemerkenswerten Punkten des Zahnorgans und/oder zwischen einem bemerkenswerten Punkt des Zahnorgans und einem anderen Punkt.

2. Trainingsverfahren nach dem unmittelbar vorstehenden Anspruch, wobei das neuronale Netz faltend ist.

3. Trainingsverfahren nach einem der vorstehenden Ansprüche, wobei
- das Zahnorgan ein Zahn oder ein Satz von weniger als 5 Zähnen ist; und/oder
- das räumliche Attribut weniger als 30 Variablen aufweist; und/oder
- der Satz historischer Bilder jedes historischen Datensatzes mehr als zwei und weniger als 30 historische Bilder aufweist; und/oder
- jedes historische Bild des Satzes historischer Bilder jedes historischen Datensatzes eine Winkelung aufweist, die aus einer Gruppe möglicher Winkelungen ausgewählt ist, die mehr als 2 und weniger als 30 mögliche Winkelungen aufweist, und/oder
- mindestens drei historische Bilder jedes Satzes historischer Bilder unterschiedliche Winkel vorweisen.

4. Trainingsverfahren nach einem der vorstehenden Ansprüche, wobei das historische Zahnorgan ein Zahn ist, der eine zuvor bestimmte Anzahl oder ein Satz von Zähnen besitzt, der einen Zahn besitzt, der eine zuvor bestimmte Anzahl und einen oder zwei Zähne aufweist, die an den Zahn angrenzen.

5. Trainingsverfahren nach einem der vorstehenden Ansprüche, wobei die historischen Bilder echte Farbfotos sind und/oder keinen Zahnspreizer darstellen und/oder extraoral sind.

6. Computerimplementiertes Verfahren für die Analyse der Zahnsituation eines Patienten, genannt "aktualisierter Patient", zu einem aktualisierten Zeitpunkt, wobei das Verfahren die folgenden Schritte aufweist:
a) vor dem aktualisierten Zeitpunkt, Trainieren eines neuronalen Netzes im Anschluss an ein Trainingsverfahren nach einem der vorstehenden Ansprüche;
b) zu dem aktualisierten Zeitpunkt, Erfassen, mittels einer Bilderfassungsvorrichtung, eines Satzes aktualisierter Bilder, die mit dem neuronalen Netz kompatibel sind und das aktualisierte Zahnorgan des Patienten darstellen, genannt "aktualisiertes Zahnorgan";
c) Analysieren des Satzes aktualisierter Bilder mittels des neuronalen Netzes, um eine räumliche Information zu erhalten, die für den aktualisierten Patienten einen Satz von Werten für das räumliche Attribut aufweist, genannt "aktualisierte räumliche Information".

7. Analyseverfahren nach dem unmittelbar vorstehenden Anspruch, wobei die aktualisierten Bilder echte Farbfotos und/oder extraoral sind und/oder bei dem in Schritt b) die Bilderfassungsvorrichtung ein Mobiltelefon ist und/oder der aktualisierte Patient keinen Zahnspreizer trägt.

8. Analyseverfahren nach einem der zwei unmittelbar vorstehenden Ansprüche, wobei in Schritt b) der aktualisierte Patient einen Zahnspreizer trägt.

9. Analyseprogramm nach einem der drei unmittelbar vorstehenden Ansprüche, wobei
- in Schritt a) eine Vielzahl von neuronalen Netzen mit jeweiligen historischen Lernbasen trainiert wird, die sich darin unterscheiden, dass sie unterschiedliche Zahnorgane und/oder unterschiedliche räumliche Attribute betreffen, um so eine Vielzahl von spezialisierten neuronalen Netzwerken zu erhalten;
- in Schritt b) aktualisierte Bilder erfasst werden und aus den aktualisierten Bildern für jedes der spezialisierten neuronalen Netze ein Satz aktualisierter Bilder erzeugt wird;
- in Schritt c) jeder Satz aktualisierter Bilder mittels des entsprechenden spezialisierten neuronalen Netzes analysiert wird, um eine Vielzahl aktualisierter räumlicher Informationen zu erhalten.

10. Analyseverfahren nach dem unmittelbar vorstehenden Anspruch, wobei in Schritt a) jede historische Lernbasis einen Zahn, der eine für die historische Lernbasis spezifische Nummer besitzt, oder eine Gruppe von Zähnen betrifft, wobei die Nummern der Zähne der Gruppe für die historische Lernbasis spezifisch sind.

11. Analyseverfahren nach einem der fünf unmittelbar vorstehenden Ansprüche, wobei die aktualisierte räumliche Information zum Bewerten, ob ein Ziel erreicht wird, und/oder zum Messen des Unterschieds zwischen der Zahnsituation des aktualisierten Patienten zu dem aktualisierten Zeitpunkt und der Verwirklichung des Ziels verwendet wird, wobei das Ziel aus den folgenden Zielen ausgewählt ist:
- der aktualisierte Patient erreicht Okklusionsklasse Nr. I für die Eckzähne,
- der aktualisierte Patient erreicht Okklusionsklasse Nr. I für die Backenzähne,
- die Zwischenräume des vorderen Sektors des aktualisierten Patienten sind geschlossen,
- der Zwischenraum, der durch die Extraktion eines Zahns des aktualisierten Patienten entsteht, ist geschlossen,
- der aktualisierte Patient weist einen normalen horizontalen Overjet vor,
- der aktualisierte Patient weist einen normalen vertikalen Overjet vor,
- die interinzisalen Mittelpunkte des Ober- und des Unterkiefers des aktualisierten Patienten sind nicht versetzt,
- der aktualisierte Patient weist keinen seitlichen Versatz des Unterkieferbogens und/oder des Oberkieferbogens im Verhältnis zu einer Sagittalebene des Kopfes des Patienten vor,
- der aktualisierte Patient weist keinen seitlichen Versatz des oberen Zahnbogens im Verhältnis zu dem unteren Zahnbogen vor,
- eine kieferorthopädische Vorrichtung, die durch den Patienten getragen wird, trägt nicht mehr zum Verändern der Positionen der Zähne des aktualisierten Patienten bei,
- keine oder nur geringe Zahnbewegungen des aktualisierten Patienten werden bei der oder den zwei letzten Kontrollen des Ober- und/oder Unterkiefers erkannt,
- alle provisorischen Zähne des aktualisierten Patienten sind ausgefallen,
- Fehlen eines seitlichen offenen Bisses,
- Fehlen eines hinteren offenen Bisses,
- Fehlen eines vorderen offenen Bisses,
- Fehlen einer umgekehrten vorderen Artikulation,
- Fehlen einer umgekehrten hinteren Artikulation,
- Verbesserung des Engstands,
- Stabilisierung von Rezessionen,
- geschlossene Zahnlücken,
- Fehlen von Unregelmäßigkeiten der Schleimhaut.

12. Computerprogramm, umfassend Codeanweisungen zum Implementieren der Schritte eines Verfahrens zum Bestimmen eines Bewegungsausmaßes zwischen einem "früheren" aktualisierten Zeitpunkt und einem "späteren" aktualisierten Zeitpunkt nach dem früheren aktualisierten Zeitpunkt, wobei das Verfahren die folgenden Schritte aufweist:
1) Implementieren eines Analyseverfahrens nach einem der Ansprüche 6 bis 11 zu dem vorherigen aktualisierten Zeitpunkt, um eine vorherige aktualisierte räumliche Information zu erhalten;
2) Implementieren eines Analyseverfahrens nach einem der Ansprüche 6 bis 11 zu einem späteren aktualisierten Zeitpunkt, um später aktualisierte räumliche Informationen zu erhalten;
3) Vergleichen der früheren und der späteren aktualisierten räumlichen Information, um ein Bewegungsausmaß zwischen dem früheren und dem späteren aktualisierten Zeitpunkt zu erhalten;
4) optional Präsentieren des Bewegungsausmaßes an den aktualisierten Patienten und/oder an eine Zahnpflegefachperson.

13. Computerprogramm nach dem unmittelbar vorstehenden Anspruch, wobei das Bewegungsausmaß eine Amplitude und/oder eine Geschwindigkeit der Verschiebung in Translation und/oder Rotation zwischen dem vorherigen und dem späteren aktualisierten Zeitpunkt eines oder mehrerer Punkte des aktualisierten Zahnorgans und/oder eines oder mehrerer Vektoren definiert, die Punkte des aktualisierten Zahnorgans verbinden oder einen oder mehrere Punkte des aktualisierten Zahnorgans und einen oder mehrere andere Punkte der Mundhöhle des aktualisierten Patienten verbinden.

14. Computerprogramm nach einem der zwei unmittelbar vorstehenden Ansprüche, wobei in Schritt 3) das Bewegungsausmaß mit einem Schwellenwert verglichen wird und, in Abhängigkeit von dem Unterschied zwischen dem Bewegungsausmaß und dem Schwellenwert,
- ein aktueller Aktivitätsindex einer kieferorthopädischen Vorrichtung, die durch den aktualisierten Patienten getragen wird, und/oder
- ein Übereinstimmungsindex der Zahnsituation des aktualisierten Patienten mit einer Situation, die durch eine kieferorthopädische Behandlung vordefiniert ist, die durch den aktualisierten Patienten durchlaufen wird, oder mit einer Situation, die aus einer kieferorthopädischen Behandlung entsteht, die durch den aktualisierten Patienten durchlaufen wird, oder, unabhängig von der kieferorthopädischen Behandlung, mit einer Situation, die durch eine Zahnpflegefachperson definiert wird, bestimmt wird.

15. Computerprogramm nach einem der drei unmittelbar vorstehenden Ansprüche, wobei der Aktivitätsindex und/oder der Übereinstimmungsindex in grafischer Form dargestellt werden.

16. Verwendung eines Analyseverfahrens nach einem der Ansprüche 6 bis 11 zum:
- Erkennen oder Bewerten der Position oder Form eines Zahns, und/oder
- Erkennen oder Bewerten einer Position oder Form einer kieferorthopädischen Vorrichtung, und/oder
- in der Zahnmedizin.

17. Verwendung nach dem unmittelbar vorstehenden Anspruch zum
- Erkennen eines Rezidivs oder einer abnormalen Position eines Zahns, und/oder
- Erkennen einer Abrasion eines Zahns, und/oder
- Kontrollieren der Stabilität oder Veränderung der Okklusion,
- Erkennen oder Bewerten einer Ablösung eines Rings oder einer kieferorthopädischen Schiene,
- Optimieren des Terminvereinbarungsdatums mit einem Kieferorthopäden oder Zahnarzt, und/oder
- Bewerten der Wirksamkeit einer aktiven kieferorthopädischen Behandlung, und/oder
- Messen der Aktivität einer aktiven kieferorthopädischen Vorrichtung; und/oder
- Messen des Verlusts der Wirksamkeit einer passiven kieferorthopädischen Vorrichtung.

## Claims

1. A computer-implemented method for training a neural network intended for analyzing a dental situation of an updated patient, said method comprising the following steps:
A) creating a historical learning base relating to a dental organ and to a spatial attribute associated with the dental organ, the historical learning base comprising more than 1000 historical records, each historical record, relating to a respective historical patient, comprising:
- a set of historical images all representing said dental organ of said historical patient, referred to as the "historical dental organ"; and
- spatial information comprising, for the historical patient, a set of values for said spatial attribute, referred to as "historical spatial information";
B) training the neural network, providing it with said sets of historical images as input and said historical spatial information as output, the spatial attribute defining an ordered sequence of variables in a three-dimensional reference frame, the spatial attribute defining:
- a position of one or more remarkable points of the dental organ in a three-dimensional reference frame; and/or
- one or more vectors between remarkable points of the dental organ and/or between a remarkable point of the dental organ and another point.

2. . The training method according to the immediately preceding claim, wherein the neural network is convolutional.

3. . The training method according to any one of the preceding claims, wherein
- the dental organ is a tooth or a set of less than 5 teeth; and/or
- the spatial attribute comprises less than 30 variables; and/or
- the set of historical images of any historical record comprises more than two and less than 30 historical images; and/or
- each historical image of the set of historical images of any historical record has an angulation selected from a group of potential angulations comprising more than 2 and less than 30 potential angulations, and/or
- at least three historical images of any set of historical images have different angulations.

4. . The training method according to any one of the preceding claims, wherein the historical dental organ is a tooth having a predetermined number or a set of teeth comprising a tooth having a predetermined number and one or two teeth adjacent to said tooth.

5. . The training method according to any one of the preceding claims, wherein the historical images are real color photographs, and/or do not represent a dental retractor, and/or are extra-oral.

6. . A computer-implemented method for analyzing a dental situation of a patient, referred to as an "updated patient", at an updated time, said method comprising the following steps:
a) prior to the updated time, training a neural network in accordance with a training method according to any one of the preceding claims;
b) at the updated time, acquiring, by means of an image acquisition apparatus, a set of updated images compatible with said neural network and representing said dental organ of the updated patient, referred to as the "updated dental organ";
c) analyzing the set of updated images by means of said neural network so as to obtain spatial information comprising, for the updated patient, a set of values for said spatial attribute, referred to as "updated spatial information".

7. . The analysis method according to the immediately preceding claim, wherein the updated images are real color, and/or extra-oral photographs and/or wherein, in step b), the image acquisition apparatus is a cell phone, and/or the updated patient is not wearing a dental retractor.

8. . The analysis method according to any one of the two immediately preceding claims, wherein, in step b), the updated patient wears a dental retractor.

9. . An analysis method according to any one of the three immediately preceding claims, wherein
- in step a), a plurality of neural networks are trained with respective historical training bases which differ in that they relate to different dental organs and/or different spatial attributes, so as to obtain a plurality of specialized neural networks;
- in step b), updated images are acquired and, from said updated images, a set of updated images is generated for each of said specialized neural networks;
- in step c), each said set of updated images is analyzed by means of the corresponding specialized neural network, so as to obtain a plurality of updated spatial information.

10. . The analysis method according to the immediately preceding claim, wherein, in step a), each historical learning base relates to a tooth having a number specific to said historical learning base, or a group of teeth, the numbers of the teeth of said group being specific to said historical learning base.

11. . The analysis method according to any one of the five immediately preceding claims, wherein the updated spatial information is used to evaluate whether a goal is achieved and/or to measure the difference between the dental situation of the updated patient at the updated time and the achievement of the goal, the goal being selected from the following goals:
- the updated patient has reached occlusion class I for canines,
- the updated patient has reached occlusion class I for molars,
- the spaces in the anterior sector of the updated patient are closed,
- the space resulting from the extraction of a tooth from the updated patient is closed,
- the updated patient has a normal horizontal overlap,
- the updated patient has a normal vertical overlap,
- the interincisal midlines of the upper and lower arches of the updated patient are not offset,
- the updated patient shows no lateral shift of the lower arch and/or upper arch with respect to a sagittal plane of the patient's head,
- the updated patient has no lateral shift of the upper arch with respect to the lower arch,
- an orthodontic apparatus worn by the updated patient no longer acts to modify the positions of the teeth of the updated patient,
- little or no movement of the teeth of the updated patient detected during the last one or two checks of the upper and/or lower arch,
- all the temporary teeth of the updated patient have fallen out,
- no lateral open bite,
- no posterior open bite,
- no anterior open bite,
- no anterior underbite,
- no posterior underbite,
- improvement in crowding,
- stabilization of recessions,
- diastemas closed,
- no mucosal irregularities.

12. . A computer program comprising code instructions for implementing the steps of a method for determining an amount of movement between an "earlier" updated time and a "later" updated time subsequent to the earlier updated time, said method comprising the following steps:
1) implementing an analysis method according to any one of claims 6 to 11, at the earlier updated time, so as to obtain earlier updated spatial information;
2) implementing an analysis method according to any one of claims 6 to 11, at the later updated time, so as to obtain later updated spatial information;
3) comparing earlier and later updated spatial information to obtain an amount of movement between the earlier and later updated times;
4) optionally, presenting said amount of movement to the updated patient and/or to a dental care professional.

13. . The computer program according to the immediately preceding claim, wherein the amount of movement defines an amplitude and/or a speed of translational and/or rotational displacement, between the earlier and later updated times, of one or more points of the updated dental organ and/or of one or more vectors connecting points of the updated dental organ or connecting one or more points of the updated dental organ and one or more other points of the oral cavity of the updated patient.

14. . The computer program according to any one of the two immediately preceding claims, wherein in step 3), said amount of movement is compared with a threshold value and, depending on the difference between the amount of movement and the threshold value,
- an activity index for an orthodontic apparatus worn by the updated patient and/or
- an index of compliance of the dental situation of the updated patient to a situation predefined by an orthodontic treatment undergone by the updated patient, or to a situation resulting from an orthodontic treatment undergone by the updated patient, or, independently of an orthodontic treatment, to a situation defined by a dental care professional is determined.

15. . The computer program according to any one of the three immediately preceding claims, wherein the activity index and/or the compliance index are presented in graphical form.

16. . Use of an analysis method according to any one of claims 6 to 11 for:
- detecting or evaluating a position or a shape of a tooth, and/or
- detecting or evaluating a position or a shape of an orthodontic apparatus, and/or
- in dentistry.

17. . The use according to the immediately preceding claim, for
- detecting a recurrence or an abnormal position of a tooth, and/or
- detecting an abrasion of a tooth, and/or
- checking the stability or the modification of the occlusion,
- detecting or evaluating a detached orthodontic band or aligner,
- optimizing the appointment date with an orthodontist or a dentist, and/or
- evaluating the effectiveness of an active orthodontic treatment, and/or
- measuring the activity of an active orthodontic apparatus; and/or
- measuring a loss of efficiency of a passive orthodontic apparatus.
